(19) Europäisches Patentamt
European Patent Office
Office européen des brevets

(11) EP 4 281 538 B1

# (12) EUROPEAN PATENT SPECIFICATION

(45) Date of publication and mention of the grant of the patent:
**22.04.2026 Bulletin 2026/17**

(21) Application number: **22743433.9**

(22) Date of filing: **25.01.2022**

(51) International Patent Classification (IPC):
***C12M 1/00*** *(2006.01)*

(52) Cooperative Patent Classification (CPC):
**B01L 3/502; C12N 1/20; C12Q 1/24;**
B01L 2200/0684; B01L 2300/042; B01L 2300/0645;
B01L 2300/069

(86) International application number:
**PCT/US2022/070339**

(87) International publication number:
**WO 2022/159989 (28.07.2022 Gazette 2022/30)**

## (54) APPARATUS, SYSTEMS, AND METHODS FOR PREPARING AN OUTPUT SAMPLE WITH AERATION

VORRICHTUNG, SYSTEME UND VERFAHREN ZUR HERSTELLUNG EINER AUSGANGSPROBE MIT BELÜFTUNG

APPAREIL, SYSTÈMES ET PROCÉDÉS DE PRÉPARATION D’UN ÉCHANTILLON DE SORTIE PRÉSENTANT UNE AÉRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2021 US 202163141057 P**
**18.06.2021 US 202163212600 P**

(43) Date of publication of application:
**29.11.2023 Bulletin 2023/48**

(73) Proprietor: **Avails Medical, Inc.**
**Menlo Park, CA 94025 (US)**

(72) Inventors:
- **RAJAN, Nitin K.**
  **Palo Alto, California 94301 (US)**
- **THEISS, Andrew H.**
  **Mountain View, California 94041 (US)**
- **KNOPFMACHER, Oren S.**
  **San Francisco, California 94132 (US)**
- **HERGET, Meike**
  **Woodside, California 94062 (US)**
- **LAUFER, Michael D.**
  **Menlo Park, California 94025 (US)**
- **PUTNEY, Suzanne**
  **San Francisco, California 94107 (US)**
- **DEAK, Eszter**
  **San Jose, California 95130 (US)**

(74) Representative: **Mooser, Sebastian Thomas**
**Wuesthoff & Wuesthoff**
**Patentanwälte und Rechtsanwalt PartG mbB**
**Schweigerstraße 2**
**81541 München (DE)**

(56) References cited:
**WO-A1-03/006629**
**WO-A1-2014/207532**
**WO-A1-2019/246208**
**CN-A- 110 713 919**
**US-A1- 2011 081 713**
**US-A1- 2013 330 768**
**US-A1- 2019 293 529**
**US-A1- 2021 371 896**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 281 538 B1

## Description

### TECHNICAL FIELD

The present disclosure relates generally to preparation of diagnostic samples and, more specifically, to apparatus, systems, and methods for preparing an output sample of bacteria of a target or desired concentration (or within acceptable error margins thereof) using ORP monitoring and aeration.

### BACKGROUND

Infections caused by anti-infective resistant bacteria are a significant problem for healthcare professionals in hospitals, nursing homes, and other healthcare environments. Rapid detection of the susceptibility of such bacteria to antibiotics is crucial in order to prevent the spread of their resistance profiles. While new technologies (e.g., matrix-assisted laser desorption/ionization-time of flight mass spectrometry (MALDI-TOF MS), rapid polymerase chain reaction (rapid PCR), etc.) have been developed for identifying bacteria in samples such as positive blood cultures, the first step in most antibiotic susceptibility testing (AST) protocols still involves preparation of an output sample or inoculum having a concentration that matches a McFarland standard.

Existing methods and instruments used to prepare such output samples include costly, time-intensive (e.g., up to 24 hours), and labor-intensive microbial culturing techniques. However, those methods often require manual interpretation by skilled personnel and are prone to technical or clinician error. In addition, certain samples containing animal or human blood are often difficult to assess using prevailing optical techniques given the samples' opacity. Moreover, such optical techniques often require expensive equipment. Furthermore, while some methods discuss using a universal look-up table (LUT) to prepare an output sample, one drawback of methods that rely solely on a universal LUT is that the time to reach the target concentration is heavily influenced by the growth rate of the bacteria within the source sample. This means that the time it takes to prepare an output sample can vary greatly based on the bacteria within the source sample. This can make reliance on such methods impractical in busy laboratory settings.

As a result of the above limitations and restrictions, there is a need for improved apparatus, systems, and methods to quickly and effectively prepare an output sample of bacteria of a desired or target concentration for downstream testing.

Prior art is found in US 2021/371896 A1 which generally relates to devices, systems, and methods for preparing a standardized inoculum for antimicrobial susceptibility testing, in WO 2019/246208 A1 which generally relates to devices, systems, and methods for measuring a solution characteristic of a sample comprising microorganisms, in US 2019/293529 A1 which generally relates to an apparatus, systems, and methods for preparing an output sample comprising a defined concentration of an infectious agent for downstream testing, in CN 110 713 919 A which generally relates to a marine algae culture device and application thereof, in WO 2014/207532 A1 which generally relates to a positive-displacement micropump, in US 2013/330768 A1 which generally relates to methods for culturing human myeloid leukemia cells and cells derived therefrom, in US 2011/081713 A1 which generally relates to a system and apparatus for feeding, solubilizing, growing and discharging a biological material, and in WO 03/006629 A1 which generally relates to a culturing method and device for photosynthetic microbes.

### SUMMARY

The invention is set out in the independent claim. Preferred embodiments of the invention are set out in the dependent claims.

According to a first aspect, we describe a method of preparing a sample of bacteria of a desired or target concentration or within acceptable error margins of the desired or target concentration, comprising: introducing an aliquot of a sample comprising the bacteria into a sample container, wherein the aliquot of the sample within the sample container is a contained sample in fluid communication with a reference sensor and an active sensor; incubating and aerating the contained sample, wherein the contained sample is aerated at a flow rate of between 7.0 microliter, μL, per second per milliliter, mL, of the contained sample and 10.0 μL per second per mL of the contained sample; monitoring a change in an oxidation reduction potential, ORP, of the contained sample using a reader electrically coupled to the reference sensor and the active sensor; and cooling the contained sample when a concentration of the bacteria in the contained sample is determined to have reached the desired or target concentration or within acceptable error margins thereof.

In some examples, the method further comprises retrieving a species-agnostic look-up table, LUT, from a database, wherein the species-agnostic LUT comprises species-agnostic ORP change amounts associated with species-agnostic bacterial concentrations, wherein the species-agnostic LUT is generated from a plurality of constituent LUTs comprising ORP change amounts and bacterial concentrations measured using a plurality of reference bacterial samples incubated and aerated at a flow rate of between 7.0 μL per second per mL of each of the reference bacterial samples and

10.0 μL per second per mL of each of the reference bacterial samples.

In some examples, the method further comprises selecting one of the species-agnostic ORP change amounts as a threshold ORP change amount when the species-agnostic ORP change amount selected is associated with one of the species-agnostic bacterial concentrations equal to the desired or target concentration; and determining that the concentration of the bacteria in the contained sample has reached the desired or target concentration or within acceptable error margins thereof when the change in the ORP of the contained sample monitored by the reader reaches the threshold ORP change amount.

In some examples, the species-agnostic LUT can be generated from at least three constituent LUTs including a first LUT, a second LUT, and a third LUT; wherein each of the first LUT, the second LUT, or the third LUT is either a species-specific LUT or a strain-specific LUT. The first LUT, the second LUT, and the third LUT can be generated using ORP measurements and bacterial concentration measurements made of a first reference bacterial sample, a second reference bacterial sample, and a third reference bacterial sample, respectively. The first reference bacterial sample can comprise a bacteria of a first species, the second reference bacterial sample can comprise a bacteria of a second species different from the first species, and the third reference bacterial sample can comprise a bacteria of a third species different from the second species and the first species.

In some examples, each of the strain-specific LUTs can be generated by: monitoring a change in the ORP of at least one reference bacterial sample over a period of time; periodically conducting optical density, OD, measurements of the at least one reference bacterial sample over the same period of time; converting results of the OD measurements to reference sample bacterial concentrations using a conversion factor; and associating the reference sample bacterial concentrations with the change in the ORP of the at least one reference bacterial sample.

In some examples, the method further comprises calculating a time-to-target concentration, $t_{target}$, representing an amount of time required for the contained sample to reach the desired or target concentration, $N_{target}$, of bacteria using the following relationship:

$$t_{target} = t_1 + \left(t_{doubling_average} \times \log_2\left(\frac{N_{target}}{N_1}\right)\right)$$

wherein $N_{target}$ is not included in the species-agnostic LUT and $N_1$ is a species-agnostic bacterial concentration included in the species-agnostic LUT, wherein $t_1$ represents a time required for the ORP of the contained sample to change by a species-agnostic ORP change amount, $\Delta_{ORP}$, associated with $N_1$ from the species-agnostic LUT, and wherein $t_1$ is determined from real-time ORP monitoring conducted by the reader on the contained sample, and wherein $t_{doubling_average}$ is an average bacterial doubling time. The method can also comprise determining that the concentration of the bacteria in the contained sample has reached the desired or target concentration or within acceptable error margins thereof when a time elapsed equals the time-to-target concentration.

In some examples, the method further comprises calculating a time-to-target concentration, $t_{target}$, representing an amount of time required for the contained sample to reach the desired or target concentration, $N_{target}$, of bacteria using the following relationship:

$$t_{target} = t_1 + \left(t_{doubling_average} \times \log_2\left(\frac{N_{target}}{N_1}\right)\right)$$

wherein $N_{target}$ and $N_1$ are both included in the species-agnostic LUT, wherein $N_{target}$ is greater than $N_1$, $N_{target} > N_1$, wherein $t_1$ represents a time required for the ORP of the contained sample to change by a species-agnostic ORP change amount, ΔORP, associated with $N_1$ from the species-agnostic LUT, and wherein $t_1$ is determined from real-time ORP monitoring conducted by the reader on the contained sample, and wherein $t_{doubling_average}$ is an average bacterial doubling time. The method can also comprise determining that the concentration of the bacteria in the contained sample has reached the desired or target concentration or within acceptable error margins thereof when a time elapsed equals the time-to-target concentration.

In some examples, the output sample can be prepared without any prior knowledge of a species of the bacteria in the contained sample or previously ascertaining a species of the bacteria in the contained sample. In some examples, the bacteria within the contained sample can be a facultative anaerobe or a strict aerobe. In some examples, the desired or target concentration can be between $1.4 \times 10^8$ CFU/mL and $1.6 \times 10^8$ CFU/mL.

In some examples, the method further comprises diluting a source sample comprising the bacteria by a dilution factor between 1:10 and 1:100 to yield a diluted sample, wherein the aliquot of the sample introduced into the sample container can be an aliquot of the diluted sample.

In some examples, the reference sensor comprises a reference electrode material and a wick in fluid communication with the contained sample such that least some of the contained sample within a chamber cavity of the sample container is drawn by the wick in a direction of the reference electrode material and the contained sample is in fluid contact with the reference electrode material, wherein the active sensor is coupled to at least part of a chamber lateral wall of the sample container, wherein an active electrode material of the active sensor faces the chamber cavity such that the contained sample is in fluid contact with the active electrode material when the contained sample fills the chamber cavity, and wherein the ORP of the contained sample is determined by the reader based on a potential difference measured between the active electrode material and the reference electrode material when the reference sensor and the active sensor are electrically coupled to the reader.

In some examples, the contained sample can be aerated in accordance with an aeration cycle, wherein the aeration cycle can comprise an aeration period followed by a non-aerated period, wherein the aeration period can be longer than the non-aerated period, and wherein the the aeration period can be between about 7 minutes and 10 minutes and the non-aerated period can be between about 3 seconds and 10 seconds.

In some examples, the contained sample can be aerated using a motorized piston pump, wherein the motorized piston pump can be housed within the reader, and wherein aerating the contained sample can further comprise pumping ambient air into the sample container through an opening defined along a base of the sample container.

## BRIEF DESCRIPTION OF THE DRAWINGS

The embodiments of figures 1, 2, and 5 to 8A are not according to the present invention, and are present for illustration purposes only. Additionally, non-method embodiments are not according to the present invention, and are present for illustration purposes only.

Fig. 1A illustrates a front view of one embodiment of a sensor apparatus for preparing an output sample of bacteria of a desired or target concentration (or within acceptable error margins of the desired or target concentration) using ORP monitoring and aeration.

Fig. 1B illustrates a cross-sectional side view of part of the sensor apparatus.

Fig. 1C illustrates a perspective close-up view of an active sensor adhered to a chamber lateral wall of the sensor apparatus.

Fig. 1D illustrates a sectional view of a sample-filled sensor apparatus.

Fig. 2A illustrates one embodiment of a reader that can be used to monitor the ORP of a sample contained within the sensor apparatus.

Fig. 2B illustrates certain functional components of the reader with a reader housing removed for use of viewing.

Fig. 2C illustrates a partial side cross-sectional view of the sensor apparatus positioned within the reader.

Fig. 3 illustrates one embodiment of a method of preparing an output sample of bacteria of a desired or target concentration (or within acceptable error margins of the desired or target concentration).

Fig. 4 illustrates one embodiment of a species-agnostic look-up table (LUT) generated from multiple constituent LUTs.

Fig. 5 illustrates one embodiment of a species-agnostic LUT generated from six strain-specific LUTs.

Figs. 6A and 6B illustrate results from 41 trial runs that were performed to evaluate the efficacy of the method and system disclosed herein for preparing an output sample of bacteria of a desired or target concentration (or within acceptable error margins of the desired or target concentration).

Figs. 7A and 7B are graphs illustrating the effect of aeration on the bacterial growth rates of a facultative anaerobe and a strict aerobe, respectively.

Fig. 8A is a table showing that aeration can reduce the variance in the growth rates of different species of bacteria.

Fig. 8B is a table showing that an average bacterial doubling time can calculated from a plurality of species-specific bacterial doubling times.

Fig. 9A is an ORP growth curve illustrating the change in the ORP of a contained sample measured by the reader over a period of time.

Fig. 9B is a bacterial growth curve illustrating a change in the bacterial concentration of the contained sample over a period of time.

## DETAILED DESCRIPTION

Variations of the apparatus, devices, systems, and methods disclosed herein are best understood from the detailed description when read in conjunction with the accompanying drawings. It is emphasized that, according to common practice, the various features of the drawings may not be to scale. On the contrary, the dimensions of the various features may be arbitrarily expanded or reduced for clarity and not all features may be visible or labeled in every drawing. The drawings are taken for illustrative purposes only and are not intended to define or limit the scope of the claims to that which is shown.

Figs. 1A-1D illustrate one embodiment of a sensor apparatus 100 for preparing an output sample of bacteria of a desired or target concentration (or within acceptable error margins thereof) from a source sample using oxidation reduction potential (ORP) monitoring and aeration.

In certain embodiments, the source sample can comprise a bodily fluid which can be at least one of blood, urine, serum, plasma, saliva, sputum, semen, breast milk, joint fluid, spinal fluid such as cerebrospinal fluid, wound material, mucus, fluid accompanying stool, vaginal secretions, synovial fluid, pleural fluid, peritoneal fluid, pericardial fluid, and amniotic fluid.

In additional embodiments, the source sample can be a swab can be a wound swab, a rectal swab, or a vaginal swab.

In other embodiments, the source sample can be an environmental sample or a food/drink sample. For example, the source sample can comprise an environmental sample obtained from a stream, a river, a lake, an ocean, a contamination site, a quarantine zone, an emergency area, or a combination thereof. In other embodiments, the source sample can comprise a food sample obtained from a food preparation facility, a dining establishment, or a waste facility.

In all such embodiments, the source sample can comprise or contain bacteria. In certain embodiments, the source sample can be a bacterial culture derived from at least one of a patient sample, a biological sample, an environmental sample, and a food sample. For example, the source sample can be a bacterial culture or a re-suspended bacterial culture derived from a bodily fluid (or swab).

As a more specific example, the source sample be a bacterial culture or a re-suspended bacterial culture derived from the blood of a patient or subject that has tested positive for bacterial growth. Such a source sample can also be referred to as a positive blood culture. For purposes of this disclosure, a positive blood culture (or PBC) is a bacterial culture derived from blood drawn from a patient or subject that has tested positive for bacterial growth. For example, a patient can show symptoms of sepsis (e.g., high fever, chills, etc.) and blood (e.g., 5 mL to 10 mL) can be drawn from the patient and transferred into a commercial blood culturing container or vessel that contain bacterial growth media (e.g., 30 mL to 40 mL of growth media). The blood culturing container or vessel can then be incubated at 35 °C $\pm$ 2 °C to allow the bacteria to proliferate. If the patient's blood is contaminated with bacteria, the bacteria will replicate within the container or vessel. A blood culturing system or apparatus can then be used to monitor for bacterial growth (such as by monitoring bacterial $CO_2$ production within the container or vessel) and the system or apparatus can determine that the sample has tested "positive" for bacterial growth when a critical $CO_2$ threshold has been met. Depending on the type of bacteria and the bacterial growth rate, the blood culture can turn positive between 7 hours and 3 days. Such a "positive blood culture" can then serve as the source sample.

As will be disclosed in more detail in the following sections, variations of the apparatus, devices, systems, and methods disclosed herein can be used to prepare an output sample or standardized inoculum of bacteria of a desired or target concentration (or within acceptable error margins thereof) from the source sample using ORP monitoring and aeration.

Fig. 1A illustrates a front view of one embodiment of the sensor apparatus 100. The sensor apparatus 100 can be designed or configured as a sample container comprising a container chamber 102 and a container cap 104 removably attached or fastened (e.g., screwed on or pressed on) to the container chamber 102.

The sensor apparatus 100 can further comprise an active sensor 106 affixed, adhered, or otherwise coupled to at least part of the container chamber 102 and a reference sensor 108 integrated into or fabricated as part of the container cap 104.

The container chamber 102 can be made in part of an inert or non-conductive material. In some embodiments, the container chamber 102 can comprise or be made in part of a polymeric material, a ceramic material or glass, or a combination thereof. As a more specific example, the container chamber 102 can comprise or be made in part of polyvinyl chloride (PVC), poly(methyl methacrylate) (PMMA), polydimethylsiloxane (PDMS), or a combination thereof

Fig. 1B illustrates a cross-sectional side view of part of the sensor apparatus 100. For ease of viewing, a reference electrode material 132 and a wicking component 134 (see, e.g., Fig. 1D) of the reference sensor 108 are not shown in Fig. 1B.

Fig. 1B illustrates that the container chamber 102 can comprise a chamber lateral wall 110 surrounding a chamber cavity 112 configured to receive and hold a contained sample 113 (see, e.g., Fig. 1D). The contained sample 113 can refer to an aliquot of the source sample that has been filtered and/or diluted and introduced into the chamber cavity 112 of the container chamber 102 (see, e.g., Fig. 3).

As shown in Fig. 1B, the active sensor 106 can be affixed, adhered, or otherwise coupled to the chamber lateral wall 110 of the container chamber 102. In other embodiments not shown in the figures, the active sensor 106 can be coupled to or otherwise positioned along a bottom of the container chamber 102.

The active sensor 106 can be coupled to at least part of the chamber lateral wall 110 at a window opening 114 defined along the chamber lateral wall 110. The chamber lateral wall 110 can comprise a recessed portion 116 surrounding the window opening 114. The recessed portion 116 can be defined along an exterior side of the chamber lateral wall 110.

Regarding placement of the active sensor 106, the active sensor 106 can be configured such that no part of the active sensor 106 extends into the chamber cavity 112, as seen in Figs. 1B and 1C. The active sensor 106 can be made of a conductive substrate covered in part by an active electrode layer 118 or active electrode material. The active electrode layer 118 of the active sensor 106 can face the chamber cavity 112 to allow the contained sample 113 within the chamber cavity 112 to be in fluid contact with the active electrode layer 118 through at least part of the chamber lateral wall 110 surrounding the window opening 114.

Fig. 1C illustrates a perspective close-up view of the active sensor 106 adhered to the chamber lateral wall 110. In the embodiment shown in Fig. 1C, the active sensor 106 is adhered to the recessed portion 116 of the chamber lateral wall 110. At least part of an active electrode layer 118 of the active sensor 106 can cover a window opening 114 defined along the chamber lateral wall 110 such that this part of the active electrode layer 118 covering the window opening 114 is positioned to be in fluid communication with the chamber cavity 112 of the container chamber 102. When the container chamber 102 is filled with the contained sample 113 (see, e.g., Fig. 1D), the contained sample 113 can make fluid contact with the portion of the active electrode layer 118 covering the window opening 114.

In some embodiments, a cavity volume of the chamber cavity 112 can be between about 0.8 mL and 1.2 mL. As a more specific example, the chamber cavity 112 can be about 1.0 mL. In other embodiments, the cavity volume of the chamber cavity 112 can be greater than 1.2 mL.

Fig. 1C also illustrates that the active sensor 106 can have its lateral sides covered by an adhesive 120. Since the active sensor 106 can comprise multiple layers, the adhesive 120 can protect certain layers of the active sensor 106 from undesired contact with the contained sample 113. The adhesive 120 can act as a barrier to prevent the contained sample 113 from contacting the lateral sides 122 of the active sensor 106. In other embodiments not shown in the figures but contemplated by this disclosure, the recessed portion 116 of the chamber lateral wall 110 can be sized such that the active sensor 106 fits tightly within the recessed portion 116 and the walls of the recessed portion 116 adjoin or bound the lateral sides 122 of the active sensor 106. This can ensure that only the exposed portion of the active electrode layer 118 contacts the contained sample 113, resulting in more accurate measurements of the solution characteristics (e.g., ORP and pH) of the contained sample 113.

To adhere the active sensor 106 to the container chamber 102, a bead of adhesive 120 can be applied to an inner ledge 124 and/or a side border 126 of the recessed portion 116 and the active sensor 106 can then be pressed into the recessed portion 116 with an end-effector of a pick-and-place machine. The active sensor 106 can be pressed or otherwise urged into the recessed portion 116 until an exterior-facing surface of the active sensor 106 is flush with an exterior surface of the chamber lateral wall 110.

The adhesive 120 can then be cured to secure the active sensor 106 in place. In some embodiments, the adhesive 120 can be a medical-grade UV-cured adhesive. The adhesive 120 can be any low-outgassing medical grade adhesive.

As previously discussed, the active sensor 106 can be made of a conductive substrate covered in part by an active electrode layer 118 or active electrode material. The active sensor 106 can be positioned such that the active electrode layer 118 faces the chamber cavity 112 to allow the sample within the chamber cavity 112 to be in fluid contact with the active electrode layer 118 through at least part of the chamber lateral wall 110 surrounding the window opening 114. In this embodiment, the active sensor 106 (including the active electrode layer 118) is positioned radially outward from an interior-facing or cavity-facing side of the chamber lateral wall 110 and the lateral sides 122 of the active sensor 106 are not exposed to the contained sample 113.

When the container chamber 102 is filled with the contained sample 113, the oxidation reduction potential (ORP) of the contained sample 113 can be measured or monitored by a reader 200 (see, e.g., Figs. 2A-2C) communicatively coupled to the sensor apparatus 100. In these embodiments, the active electrode layer 118 can be a redox-sensitive material. For example, the redox-sensitive material can be or comprise any of platinum (Pt), gold (Au), a redox sensitive metal oxide, or a combination thereof. More specifically, the redox-sensitive material can be or comprise any of silicon dioxide ($SiO_2$), aluminum oxide ($Al_2O_3$), titanium dioxide ($TiO_2$), tantalum pentoxide ($Ta_2O_5$), hafnium dioxide ($HfO_2$), iridium dioxide ($IrO_2$), ruthenium dioxide ($RuO_2$), zirconium dioxide ($ZrO_2$), or a combination thereof.

In other embodiments, the pH of the contained sample 113 can also be measured or monitored by the reader 200. When the solution characteristic of the contained sample 113 measured or monitored is pH, the active electrode layer 118 can be a pH-sensitive material. For example, the pH-sensitive material can be or comprise any of silicon dioxide ($SiO_2$), aluminum oxide ($Al_2O_3$), titanium dioxide ($TiO_2$), tantalum oxide/pentoxide ($Ta_2O_5$), hafnium dioxide ($HfO_2$), iridium dioxide ($IrO_2$), ruthenium dioxide ($RuO_2$), zirconium dioxide ($ZrO_2$), or a combination thereof.

Although not shown in the figures, it is contemplated by this disclosure that the sensor apparatus 100 can be designed such that both the pH and the ORP of the contained sample 113 are measured simultaneously. For example, the container chamber 102 of the sensor apparatus 100 can comprise multiple window openings 114 defined along the chamber lateral walls 110 of the container chamber 102. Each of these window openings 114 can then be covered by a different active sensor 106 (for example, one window opening 114 can be covered by an active sensor 106 having an active electrode layer 118 made of a redox-sensitive material and another window opening 114 can be covered by an active sensor 106 having an active electrode layer 118 made of a pH-sensitive material).

The sensor apparatus 100 can have an apparatus height. In some embodiments, the apparatus height can be between about 20.0 mm to about 50.0 mm. In other embodiments, the apparatus height can be between about 25.0 mm to about 35.0 mm. For example, the apparatus height can be about 31.3 mm.

Fig. 1D illustrates that the reference sensor 108 can be fabricated as or integrated into part of the container cap 104. The reference sensor 108 can comprise a reference conduit 128 comprising a reference conduit cavity 130 (see Fig. 1B). The reference conduit cavity 130 can have first and second openings at opposite ends of the reference conduit cavity 130. The reference conduit 128 can be an elongate channel or passageway configured to extend into the chamber cavity 112 of the container chamber 102.

The reference sensor 108 can also comprise a reference electrode material 132 and a wick or wicking component 134 in fluid communication with the chamber cavity 112. The reference conduit cavity 130 can house the wicking component 134. At least some of the contained sample 113 can be drawn up by the wicking component 134 in a direction of the reference electrode material 132.

The reference conduit 128 can be tapered such that a volume of the reference conduit cavity 130 tapers or narrows from a reference conduit proximal end 136 to a reference conduit distal end 138 (see Fig. 1B). The shape of the wicking component 134 can match or accommodate the shape of the reference conduit cavity 130. The wicking component 134 can be configured such that the shape of the wicking component 134 tapers or narrows from a wick proximal end 140 to a wick distal end 142.

The wicking component 134 can extend through a length of the reference conduit cavity 130. In some embodiments, the wicking component 134 can fill up or occupy all of the space within the reference conduit cavity 130. In other embodiments, the wicking component 134 can partially fill up or partially occupy the space within the reference conduit cavity 130.

At least part of the wicking component 134 can be in fluid communication with the chamber cavity 112 of the container chamber 102 such that when the container chamber 102 is filled with the contained sample 113, at least some of the contained sample 113 in the container chamber 102 is drawn up, absorbed, or otherwise wicked by at least a portion of the wick distal end 142 in a direction of the wick proximal end 140. The wicking component 134 can be made of a polymeric material that draws up the contained sample 113 towards the reference electrode material 132 by capillary action.

In some embodiments, at least part of the wick distal end 142 can extend past the reference conduit distal end 138 such that the wick distal end 142 protrudes or extends into the chamber cavity 112 of the container chamber 102. In these embodiments, the wick distal end 142 can extend or protrude into the contained sample 113 when the container chamber 102 is filled by the contained sample 113.

In other embodiments, the wick distal end 142 is positioned proximal or above the reference conduit distal end 138 such that the wick distal end 142 does not protrude or extend into the chamber cavity 112 of the container chamber 102. In these embodiments, the wick distal end 142 can still be in fluid communication with the container chamber 102 and the contained sample 113 can still reach or contact the wick distal end 142 by being drawn up into the reference conduit 128 by capillary action or by perturbing or shaking the container chamber 102.

As previously discussed, the wicking component 134 can be made in part of a porous material. The wicking component 134 can be made in part of a material comprising pores sized between 15 $\mu$m to about 150 $\mu$m (e.g., about 50 $\mu$m). In some embodiments, the wicking component 134 can be made in part of a polymeric material. As a more specific example, the wicking component 134 can be made in part of a porous polymeric material comprising pores sized between 15 $\mu$m to about 150 $\mu$m. In one embodiment, the wicking component 134 can be made in part of high-density polyethylene (HDPE). For example, the wicking component 134 can be made in part of HDPE having pores sized about 50 $\mu$m. In other embodiments, the wicking component 134 can be made in part of natural fibers. For example, the wicking component 134 can be made in part of cellulose fibers, pulp, paper, cotton, or a combination thereof.

The wicking component 134 can also be treated by a surfactant such that at least a surface of the wicking component 134 is covered by the surfactant. In some embodiments, the wicking component 134 can be saturated by the surfactant or immersed in a solution comprising the surfactant prior to being introduced into the reference conduit cavity 130. The surfactant can be configured to increase a hydrophilicity of the wicking component 134 (i.e., to make a substantially hydrophobic surface of the wicking component 134 more hydrophilic). In some embodiments, the surfactant can be a fluorosurfactant. In other embodiments, the surfactant can be a non-ionic surfactant such as one or more Poloxamers.

In one embodiment, the reference conduit 128 can be substantially shaped as a conic or frustoconic having a reference conduit cavity 130 also substantially shaped as a conic or frustoconic. In other embodiments, the reference conduit 128 can be substantially shaped as an elongate pyramid having a polygonal-shaped base. For example, the reference conduit 128 can be substantially shaped as an elongate triangular pyramid, square pyramid, or a pentagonal pyramid. In additional embodiments, the reference conduit 128 can be substantially shaped as a cylinder having a substantially cylindrical-shaped reference conduit cavity 130. In these embodiments, the reference conduit 128 can have a tapered reference conduit distal end 138 (see, e.g., Fig. 1B).

As shown in Fig. 1D, at least part of the wicking component 134 can be in fluid contact with the contained sample 113 in the container chamber 102. At least some of the contained sample 113 can be drawn up by the wicking component 134 in a direction of the wick proximal end 140. The reference electrode material 132 can be disposed at the wick proximal end 140.

Fig. 1D also illustrates that at least part of the active electrode layer 118 can be in fluid contact with the contained sample 113 in the container chamber 102. When the wicking component 134 draws or wicks up the contained sample 113, the contained sample 113 can reach the reference electrode material 132 and charge carriers within the contained sample 113 can establish an electrical connection between the reference electrode material 132 of the reference sensor 108 and the active electrode layer 118 of the active sensor 106. When both the reference sensor 108 and the active sensor 106 are electrically coupled to a reader 200 (see, e.g., Figs. 2A-2C), the reader 200 can be used to measure a solution characteristic (e.g., ORP or pH) of the contained sample 113.

The solution characteristic (e.g., ORP or pH) of the contained sample 113 can be determined based on a potential difference measured between the active sensor 106 and the reference sensor 108 when the reference sensor 108 and the active sensor 106 are electrically coupled to the reader 200. For example, the reference sensor 108 can provide a stable half-cell potential compared to the active sensor 106 when both the reference electrode material 132 and the active electrode layer 118 are in fluid contact with the contained sample 113 within the container chamber 102.

In some embodiments, the reference electrode material 132 can be an electrically-conductive ink applied or dispensed on the wick proximal end 140. The electrically-conductive ink applied or dispensed on the wick proximal end 140 can be hardened by curing. More specifically, the electrically-conductive ink can be a silver-silver chloride (Ag-AgCl) ink.

At least part of the reference electrode material 132 can be coupled to the wicking component 134. For example, the reference electrode material 132 can be a cured and hardened mass positioned at the wick proximal end 140. In certain embodiments, the reference electrode material 132 can be positioned in the middle of the container cap 104. In some embodiments, at least part of the reference electrode material 132 can protrude or extend beyond the container cap 104.

One advantage of the wicking component 134 disclosed herein is that the wicking component 134 can draw up the sample and the contained sample 113 can advance by capillary action through the pores of the wicking component 134 toward the reference electrode material 132. For example, the contained sample 113 can be wicked to the wick proximal end 140 where it makes fluid contact with the reference electrode material 132. When the reference electrode material 132 is made of a material such as silver-silver chloride (Ag-AgCl), the wicking component 134 can act as a barrier or hindrance to silver ions ($Ag^+$) that would otherwise diffuse freely into the contained sample 113 within the container chamber 102. Such silver ions can be harmful to or otherwise affect the growth of the bacteria in the contained sample 113. The wicking component 134 can act as a barrier or hindrance to the harmful silver ions by slowing down or stalling the diffusion of such ions into the contained sample 113. The wicking component 134 having the dimensions and shape disclosed herein can be effective in slowing down or stalling the diffusion of such harmful ions.

When the reference sensor 108 is implemented as a container cap 104, the container cap 104 can have dimensions as defined by a cap width (or diameter) and a cap height. In some embodiments, the cap width can be between about 10.0 mm to about 20.0 mm. For example, the cap width can be about 15.7 mm. In some embodiments, the cap height can be between about 5.0 mm to about 20.0 mm. For example, the cap height can be about 10.5 mm. When the container cap 104 is fastened, affixed, or otherwise coupled to the container chamber 102, the sensor apparatus 100 can have an apparatus height as measured from a bottom of the container chamber 102 to a cap top 144 of the container cap 104.

The wicking component 134 can have a wick height as measured from the wick proximal end 142 to the wick distal end. In some embodiments, the wick height can be between about 10.0 mm to about 20.0 mm. More specifically, the wick height can be between about 14.0 mm to about 15.0 mm. For example, the wick height can be about 14.8 mm.

As illustrated in Fig. 1D, the reference electrode material 132 can be positioned or disposed, at least partially, within a divot, depression, or concave region in a center of the container cap 104 above the wicking component 134. When the reference sensor 108 is a cured or hardened electrically-conductive ink or solution (e.g., Ag-AgCl ink), the divot, depression, or concave region can act as a receiving space for the liquid ink or solution to be cured.

In some embodiments, the reference electrode material 132 can have a reference electrode height and a reference electrode width. The reference electrode height can be between about 0.2 mm and 1.0 mm. For example, the reference electrode height can be about 0.4 mm. The reference electrode width can be between about 2.0 mm to about 5.0 mm. For example, the reference electrode width can be about 3.0 mm. One advantage of the reference sensor 108 disclosed herein is that the reference sensor 108 can act as a stable reference electrode or provide a stable reference potential for up to 10-hours of testing or operation.

Fig. 1D also illustrates that the sensor apparatus 100 can comprise an aeration port 146 or opening defined along a bottom side of the container chamber 102. In other embodiments not shown in the figures, the aeration port 146 can be defined along the chamber lateral wall 110 of the container chamber 102.

The aeration port 146 can be covered by a first gas-permeable membrane 148. The aeration port 146 and the first gas-permeable membrane can be configured to allow a gas 150 to enter the container chamber 102.

In some embodiments, the gas 150 can be ambient air (e.g., the air in a laboratory, clinical setting, or testing facility). In other embodiments, the gas 150 can comprise a combination of pressurized oxygen, carbon dioxide, nitrogen, and argon. Aerating the sample can accelerate the growth of a bacterial population within the contained sample 113 by providing an oxygen rich environment within the container chamber 102.

In alternative embodiments not shown in the figures, the aeration port 146 can be defined along a cap top 144 of the container cap 104 and the gas 150 can be pumped into the container chamber 102 from the top of the container chamber 102.

The gas 150 (e.g., ambient air) can be pumped into the container chamber 102 by a motorized piston pump, syringe pump, or another type of pump/micropump device integrated within the reader 200. The gas 150 (e.g., ambient air) can be pumped or otherwise directed into the container chamber 102 through the aeration port 146 and the first gas-permeable membrane 148 at a flow rate of between 7.0 microliter (μL) per second per milliliter (mL) of the contained sample 113 and 10.0 μL per second per mL of the contained sample 113. As a more specific example, the gas 150 (e.g., ambient air) can be pumped or otherwise directed into the container chamber 102 through the aeration port 146 and the first gas-permeable membrane 148 at a flow rate of about 8.8 μL per second per mL of the contained sample 113. In some embodiments, the gas 150 (e.g., ambient air) can be pumped or otherwise directed into the container chamber 102 through the aeration port 146 and the first gas-permeable membrane 148 at specific duty cycles or intervals.

In certain embodiments, a second gas-permeable membrane 152 can cover at least part of an underside of the container cap 104. The second gas-permeable membrane 152 can allow any gas 150 pumped or otherwise introduced into the container chamber 102 to exit the container chamber 102 while also preventing any liquid within the container chamber 102 from spilling out of the container chamber 102.

In some embodiments, the first gas-permeable membrane 148 and the second gas-permeable membrane 152 can be made of the same material. The first gas-permeable membrane 148 and the second gas-permeable membrane 152 can be made of a hydrophobic gas-permeable film or thin-sheet. For example, the first gas-permeable membrane 148 and the second gas-permeable membrane 152 can both be made of or comprise polytetrafluoroethylene (PTFE).

As shown in Fig. 1D, the container cap 104 can be removably or detachably coupled or fastened to the container chamber 102 by being screwed on to a proximal portion of the container chamber 102 via a threaded connection 154. When the container cap 104 (serving as part of the reference sensor 108) is fastened or coupled to the container chamber 102 by the threaded connection 154, an airflow pathway 156 can be created as the gas 150 (e.g., ambient air) enters the aeration port 146 through the first gas-permeable membrane 148 into the container chamber 102. The air then exits the container chamber 102 through the second gas-permeable membrane 152 and air gaps 158 defined in between the threads of the container cap 104 and the container chamber 102.

The container cap 104 can be made in part of a transparent or clear material or a transparent or clear non-conducting material. In other embodiments, the container cap 104 can be made in part of a translucent or see-through material. For example, at least part of the wicking component 134 can be visible through the sides of the container cap 104. This can allow a user or operator of the sensor apparatus 100 to observe the wicking of the contained sample 113 from the wick distal end 142 to the wick proximal end 140 when the container cap 104 is fastened to the container chamber 102 and ensure that at least some of the contained sample 113 is able to reach the reference electrode material 132 at the wick proximal end 140. In some embodiments, the container cap 104 can be made in part of a clear or transparent polymeric material, glass, or a combination thereof.

In some embodiments, the container chamber 102, the container cap 104, or a combination thereof can be made in part of an inert polymeric material. For example, the container chamber 102, the container cap 104, or a combination thereof can be made in part of at least one of polyoxymethylene, polyamide, polyethylene, acrylonitrile butadiene styrene, polycarbonate, polypropylene, or co-polymers or composites thereof. In other embodiments, the container chamber 102, the container cap 104, or a combination thereof can be made in part a glass material such as borosilicate glass or a ceramic material.

In some embodiments, the active sensor 106 can also be insert molded into part of the chamber lateral wall 110 when the container chamber 102 is made of a polymeric material. For example, the active sensor 106 can be insert-molded into the chamber lateral wall 110 while the container chamber 102 is being formed by injection molding.

When the active sensor 106 is inserted molded into part of the chamber lateral wall 110 of the container chamber 102, the active sensor 106 can have its lateral sides 122 encapsulated by the polymeric material used to make the chamber lateral wall 110.

For example, the active sensor 106 can be insert molded such that the active electrode layer 118 faces the chamber cavity 112 to allow the contained sample 113 within the chamber cavity 112 to be in fluid contact with the active electrode layer 118 through at least part of the chamber lateral wall 110 surrounding the window opening 114.

Fig. 1D also illustrates that a side of the active sensor 106 opposite the active electrode layer 118 can be used to contact the conductive contacts or conductive connections of a reader 200 (see, e.g., Figs. 2A-2C). As will be discussed in more detail in the following sections, this side of the active sensor 106 can be referred to as a conductive layer 160.

In some embodiments, the conductive layer 160 can be a gold layer. In other embodiments, the conductive layer 160 can be made of another type of conductive metal such as platinum, nickel, copper, or alloys or composites thereof.

Although not shown in the figures, it is contemplated by this disclosure that the active sensor 106 can be affixed or otherwise coupled to the chamber lateral wall 110 by focally melting (e.g., by ultrasonic welding) a portion of the chamber lateral wall 110 surrounding the window opening 114 (see, e.g., Figs. 1B-1D for the location of the window opening 114) and pressing the active sensor 106 onto the melted portion of the chamber lateral wall 110. Once the melted portion of the chamber lateral wall 110 cools, the active sensor 106 is now affixed or coupled to the chamber lateral wall 110.

In some embodiments, the active sensor 106 can be substantially shaped as a flattened or truncated rectangular prism. In other embodiments, the active sensor 106 can be substantially disk-shaped or shaped as a flattened or truncated polygonal prism (e.g., a flattened or truncated pentagonal prism or hexagonal prism).

When the active sensor 106 is substantially shaped as a rectangular prism, the active sensor 106 can have a sensor length dimension, a sensor width dimension, and a sensor height dimension. In some embodiments, the sensor length dimension can be between about 100 $\mu$m and 6.0 mm, the sensor width dimension can be between about 100 $\mu$m and 6.0 mm, and the sensor height dimension can be between about 10 $\mu$m and 0.70 mm. For example, when the active sensor 106 is substantially shaped as a rectangular prism, the active sensor 106 can have a sensor length dimension of about 6.0 mm, a sensor width dimension of about 6.0 mm, and a sensor height dimension of about 0.61 mm.

In some embodiments, the active sensor 106 can have an active electrode layer 118 made of a noble metal. For example, the active electrode layer 118 can be made of platinum, gold, or a combination or composite thereof.

The active electrode layer 118 can be adhered to one side of a conductive substrate via an adhesion layer. The conductive substrate can be made of a conductive material such as stainless steel (SS). For example, the conductive substrate can be SS 316. In other embodiments, the conductive substrate can be made of aluminum, copper, or any combination or composite of aluminum, copper, or stainless steel.

In some embodiments, the adhesion layer can be a thin layer of chromium (Cr). Alternatively, the adhesion layer can be a thin layer of gold, nickel, titanium or tantalum. The adhesion layer can be disposed in between the conductive substrate and the active electrode layer 118.

In alternative embodiments, the active electrode layer 118 can be deposited directly onto one side of the conductive substrate without an adhesion layer.

The active electrode layer 118 can have an active electrode layer thickness of between about 50 nm and 500 nm (e.g., about 400 nm). The adhesion layer can have an adhesion layer thickness of between about 5 nm and 50 nm (e.g., about 20 nm). A ratio of the adhesion layer thickness to the active electrode layer thickness can be between about 1:10 and 1:20.

The conductive substrate can have a substrate layer thickness. The substrate layer thickness can be between about 10 $\mu$m and 0.70 mm (e.g., about 0.61 mm).

In other embodiments, the active electrode layer 118 can be made of a metal oxide. For example, the active electrode layer 118 can be made of tantalum pentoxide ($Ta_2O_5$). In other embodiments, the active electrode layer 118 can be made of silicon dioxide ($SiO_2$), silicon nitride ($Si_3N_4$), aluminum oxide ($Al_2O_3$), titanium dioxide ($TiO_2$), hafnium dioxide ($HfO_2$), iridium dioxide ($IrO_2$), ruthenium dioxide ($RuO_2$), zirconium dioxide ($ZrO_2$), or a combination or composite thereof. In these embodiments, the conductive substrate can be made of a conductive material such as stainless steel (SS). For example, the conductive material can be SS 316. The conductive substrate can also be made of aluminum, copper, or any combination or composite of aluminum, copper, or stainless steel.

The deposited layers can be selected to achieve a certain desired sensitivity or specificity towards a particular analyte. Other surface modification techniques such as self-assembled monolayers (SAMs), bio-functionalization with antibodies, binding antibody fragments, binding aptamers, binding DNA, and plasma treatments can also be employed to alter the surface properties of the deposited layers and thereby tune their specificity and sensitivity.

In certain embodiments, the active sensor 106 can leverage the scale and efficiency of printed circuit board (PCB) manufacturing techniques. For example, the active sensor 106 can be made of a non-conductive PCB substrate covered in part by an active electrode layer 118. In some embodiments, the non-conductive PCB substrate can be made of polyimide. In other embodiments, the non-conductive PCB substrate can be made of a glass-reinforced epoxy laminate material such as an FR-4 composite material. In certain embodiments, the PCB substrate can be a flexible PCB material.

In some embodiments, the active electrode layer 118 can be made of a noble metal. For example, the active electrode layer 118 can be made of platinum, gold, or a combination or composite thereof. The platinum or gold can be electrodeposited or sputter deposited on the PCB substrate.

The active electrode layer 118 can have an active electrode layer thickness of at least 50 nm. In certain embodiments, the active electrode layer 118 can have an active electrode layer thickness of at least 400 nm. When the active electrode layer 118 is made of platinum, the active sensor 106 can be used for measuring or monitoring the ORP of a sample.

In an alternative embodiment, a platinum layer deposited on the non-conductive PCB substrate can be modified with a surface modification technique to turn the platinum layer into a pH-sensitive layer. For example, an oxygen plasma treatment can be used to oxidize the platinum layer to create a platinum oxide ($PtO_2$) layer. The platinum oxide layer thus formed can respond to hydrogen ions and be used as a pH-sensitive layer. In this embodiment, the active sensor 106 can be used to measure or monitor the pH of a sample.

The PCB substrate can be patterned with conductive contacts or a conductive layer 160 on a side of the substrate opposite the active electrode layer 118. In some embodiments, the conductive layer 160 can be a gold layer. In other embodiments, the conductive layer 160 can be made of another type of conductive metal such as platinum, nickel, copper, or alloys or composites thereof.

In some embodiments, the active electrode layer 118 can be electrically coupled to the conductive layer 160 by one or more conductive vias. In one embodiment, the conductive vias can be made in part of copper or a copper alloy. In other embodiments, the conductive vias can be made of another type of conductive metal such as gold.

In some embodiments, each active sensor 106 can have at least one conductive via positioned in a center of the sensor package. In other embodiments, the conductive via can be positioned near a periphery or edge of the sensor package.

The conductive vias can be formed by electroplating, deposition, or a combination thereof. Moreover, additional features or patterns can be formed on the PCB substrate using standard PCB etching processes.

Fig. 2A illustrates one embodiment of a reader 200 configured to monitor or measure a solution characteristic (e.g., the ORP or pH) of the contained sample 113 within the container chamber 102 of the sensor apparatus 100. The reader 200 and the sensor apparatus 100 can be part of a system 301 (see, e.g., Fig. 3) for preparing an output sample of bacteria of a desired or target concentration (or within acceptable error margins thereof).

The reader 200 can comprise a reader housing 202 configured to house certain functional components of the reader 200 including a main controller 208 (see, e.g., Fig. 2B), a signal readout control unit 210 (see, e.g., Fig. 2B), a thermal control module 212 (see, e.g., Figs. 2B and 2C), and an aeration control module 214 (see, e.g., Figs. 2B and 2C). The reader housing 202 can also expose a touchscreen display 204 configured to display certain information to a user and allow the user to input commands and to input a desired or target concentration 308 (see, e.g., Fig. 3) to the reader 200. For example, the display 204 of the reader 200 can display a message or text instruction to the user that an output sample of a desired or target concentration (or within acceptable error margins thereof) has been successfully prepared (i.e., the bacteria within the contained sample 113 has reached the desired or target concentration level or has reached the desired or target concentration level within acceptable error margins thereof). Also, for example, the display 204 can display a countdown timer showing the user how much time is left before the output sample is prepared (i.e., how much time is left before the bacteria within the contained sample 113 reaches the desired or target concentration 308 or has reached a concentration level within acceptable error margins thereof).

A lid 206 or cover of the reader 200 can be opened or lifted up to reveal a container receiving space configured to accommodate or receive the sensor apparatus 100 (the container receiving space is the space occupied by the sensor apparatus 100 in Fig. 2C).

Fig. 2B illustrates certain functional components of the reader 200 with the reader housing 202 removed for ease of viewing. As shown in Fig. 2B, the reader 200 can comprise a thermal control module 212 and an aeration control module 214. The thermal control module 212 can be configured to incubate the sample-filled sensor apparatus 100. The thermal control module 212 can incubate the sensor apparatus 100 by heating at least part of the sensor apparatus 100 via a heating block 220 (see, e.g., Fig. 2C). In some embodiments, the heating block 220 can heat a lateral side of the container chamber 102 opposite the active sensor 106. In certain embodiments, the heating block 220 can partially surround or cradle the container chamber 102 to heat the sensor apparatus 100.

In some embodiments, the heating block 220 can be made in part of aluminum. In other embodiments, the heating block 220 can be made in part of another type of heat conducting metallic material.

The sensor apparatus 100 can be heated to an incubation temperature of between about 30 °C and 40 °C (e.g., about 35 °C $\pm$ 2 °C). The sensor apparatus 100 can be incubated for an incubation period. The incubation period can range from 15 minutes to over 2 hours. The incubation period can be adjusted based on the type of bacteria suspected in the source sample.

The thermal control module 212 can also be used to cool the contained sample 113 to a cooling temperature when the solution characteristic (e.g., the ORP or pH) of the contained sample 113 changes by a threshold amount indicating that the bacteria within the contained sample 113 has reached a desired or target concentration or has reached a desired or target concentration level within acceptable error margins thereof. In other embodiments, the thermal control module 212 can be used to cool the contained sample 113 to a cooling temperature when an elapsed time reaches certain time limits or time thresholds. In some embodiments, the thermal control module 212 can cool the contained sample 113 within the sensor apparatus 100 at a cooling temperature between about 4 °C and 25 °C.

When the bacteria within the contained sample 113 has reached the desired or target concentration (or within acceptable error margins thereof), the contained sample 113 within the sensor apparatus 100 can be considered an output sample ready for further downstream testing (e.g., antibiotic susceptibility testing). In certain embodiments, the reader 200 can comprise an auditory component (e.g., a speaker) and the auditory component can generate an auditory signal (i.e., sound an alarm) to notify a user or laboratory technician that the output sample has been prepared and is ready for further downstream testing.

In some embodiments, the thermal control module 212 can be controlled by the main controller 208 of the reader 200. In other embodiments, the thermal control module 212 can be controlled by another controller or module within the reader 200 or by the signal readout control unit 210.

In some embodiments, a nutrient solution or stimulus solution can be introduced into the container chamber 102 before the sensor apparatus 100 is incubated. For example, the nutrient solution can be a solution containing bacto-tryptone, yeast extract, beef extract, cation-adjusted Mueller Hinton Broth (CAMHB), starch, an acid hydrolysate of casein, calcium chloride, magnesium chloride, sodium chloride, blood or lysed blood including lysed horse blood (LHB), a CAMHB-LHB mixture, glucose, or a combination thereof. The nutrient solution can be used to counteract the buffering effects of ions or substances present in the sample when the sample is composed of a bodily fluid.

The aeration control module 214 can be configured to aerate the contained sample 113 within the container chamber 102 by pumping a gas 150 (e.g., ambient air, see Fig. 1D) into the chamber cavity 112. The gas 150 can be pumped into the container chamber 102 through an aeration port 146 defined along the bottom or base of the container chamber 102 (see also Figs. 1B and 1D).

As previously discussed, a container cap 104 (serving as part of the reference sensor 108) of the sensor apparatus 100 can be fastened or coupled to the container chamber 102 by a threaded connection 154 that allows part of an airflow pathway 156 to be created in between the threads of the container cap 104 and the threads of the container chamber 102. After the gas 150 (e.g., ambient air) enters the aeration port 146 through the first gas-permeable membrane 148 into the container chamber 102, the gas first aerates the contained sample 113 and then exits the container chamber 102 through the second gas-permeable membrane 152 and the air gaps 158 defined in between the threads of the container cap 104 and the container chamber 102.

Fig. 2C illustrates that the reader 200 can comprise a gas nozzle 222 that can be connected to the bottom of the sensor apparatus 100 to aerate the contained sample 113 within the container chamber 102. The gas nozzle 222 can be disposed at a terminal or distal end of a gas delivery conduit 224. The gas delivery conduit 224 can connect the gas nozzle 222 to the aeration control module 214. In some embodiments, at least a segment of the gas delivery conduit 224 can be positioned along or wound around a base or bottom portion of the reader 200.

In some embodiments, aeration control module 214 can comprise one or more filters (e.g., inline filters, conduit filters, pipe filters, and/or hose filters) for filtering the ambient air drawn into the aeration control module 214. In additional embodiments, the gas delivery conduit 224 can comprise an inline filter configured to filter the ambient air and remove particulates from the ambient air before the ambient air reaches the sensor apparatus 100 and/or the gas nozzle 222.

As shown in Fig. 2C, the gas nozzle 222 can connect to the aeration port 146 at the bottom of the container chamber 102 via a nozzle interface 226. In some embodiments, the nozzle interface 226 can be an O-ring. In other embodiments, the nozzle interface 226 can be another type of gasket or fluid-sealing interface.

In some embodiments, the gas 150 can be ambient air (e.g., the air in a laboratory, clinical setting, or testing facility). In other embodiments, the gas 150 can comprise a combination of pressurized oxygen, carbon dioxide, nitrogen, and argon. Aerating the sample can accelerate the growth of a microbial population within the sample by providing an oxygen rich environment within the container chamber 102.

Aerating the contained sample 113 can enhance a growth rate of the bacteria in the contained sample 113 within the sensor apparatus 100 by increasing the supply of oxygen to such bacteria. Moreover, aerating the contained sample 113 can also enable detachment of the bacteria from the interior walls of the container chamber 102 so as to inhibit biofilm formation.

Although aeration is important for enhancing the growth rate of bacteria within the sensor apparatus 100, the applicants have also discovered that too much aeration or aerating the contained sample 113 at higher flow rates can have certain detrimental effects on the ORP signal monitored by the reader 200. For example, while aerating the contained sample 113 can, in most cases, increase the growth rate of bacteria (especially aerobic bacteria) within the contained sample 113, too much aeration can suppress the ORP signal and introduce errors into the ORP measurements. Moreover, too much aeration can also cause any changes in ORP values ($\Delta_{ORP}$) to be too small to be of any value in differentiating between different bacterial concentration levels.

Furthermore, too little aeration or aerating the contained sample 113 at lower flow rates can cause the contained sample 113 to become stagnant and can cause sample preparation times to be sub-optimal or slower.

Therefore, the contained sample 113 within the sensor apparatus 100 should be aerated at a flow rate within an optimal range that avoids the aforementioned shortcomings. One such range discovered by the applicants is a flow rate between 7.0 microliter ($\mu$L) per second per milliliter (mL) of the contained sample 113 and 10.0 $\mu$L per second per mL of the contained sample 113. More specifically, the contained sample 113 can be aerated at a flow rate of about 8.8 ($\pm$0.9) $\mu$L per second per mL of the contained sample 113.

In some embodiments, the contained sample 113 can be aerated using a motorized piston pump. The motorized piston pump can be housed or contained within the reader 200. In certain embodiments, the motorized piston pump can be housed or contained completely within the reader 200. For example, the motorized piston pump can be housed or contained within the aeration control module 214.

The motorized piston pump can be actuated by one or more stepper motors and lead screw drives. The motorized piston pump can be controlled by a dedicated controller, the main controller 208, or a combination thereof. In embodiments, the contained sample 113 can be aerated using a syringe pump or a motorized syringe pump.

In some embodiments, the contained sample 113 within the sensor apparatus 100 can be aerated in accordance with an aeration cycle. The aeration cycle can include an aeration period followed by a non-aerated period where no gas or ambient air is pumped into the container chamber 102. In certain embodiments, the aeration period can be longer than the non-aerated period. For example, the aeration period can be between about 7 minutes and 10 minutes and the non-aerated period can be between about 3 seconds and 10 seconds.

One technical problem faced by the applications is that motorized piston pumps often require that the pump piston to be drawn back or re-homed once the pump piston has reached the distal end of the pump chamber or barrel. One technical solution discovered and developed by the applicants to address this technical problem is to use the non-aerated period to draw back or re-home the pump piston.

In some embodiments, the aeration control module 214 can be controlled by the main controller 208 (see, e.g., Fig. 2B). In other embodiments, the aeration control module 214 can be controlled by another controller or module within the reader 200 or by the signal readout control unit 210. For example, the amount of gas 150 (e.g., ambient air) pumped or otherwise directed into the container chamber 102 can be dictated by a change in a solution characteristic (e.g., ORP or pH) of the contained sample 113 detected by the reader 200 or the lack of any such change.

Fig. 2C also illustrates that when the sensor apparatus 100 is positioned within the container receiving space, a reference electrode contact 216 of the reader 200 can be placed or moved into contact with the reference electrode material 132 positioned on the container cap 104 (see, e.g., Fig. 1D) of the sensor apparatus 100. Moreover, when the sensor apparatus 100 is positioned within the container receiving space, an active electrode contact 218 of the reader 200 can be placed or moved into contact with a conductive layer 160 (see, e.g., Fig. 1C and 1D) or conductive contact of the active sensor 106.

In some embodiments, the reference electrode contact 216 and the active electrode contact 218 can comprise one or more conductive pogo or spring-loaded pins, conductive leaf contacts, or a combination thereof. More specifically, the conductive pogo pins or leaf contacts can be made of copper, nickel, stainless steel, or alloys thereof.

The reference electrode contact 216 and the active electrode contact 218 can be electrically coupled to a signal readout control unit 210. The signal readout control unit 210 can comprise one or more processors, chipsets, or chip modules programmed to convert and read signals obtained from the active sensor 106 and the reference sensor 108 of the sensor apparatus 100. For example, the signal readout control unit 210 can determine an ORP of the contained sample 113 within the sensor apparatus 100 based on a potential difference measured between the active electrode layer 118 and the reference electrode material 132.

The active electrode layer 118 is chosen so that it easily interacts with oxidized/reduced molecules in the contained sample 113 (i.e., no activation barrier or additional energy needs to be provided).The active electrode layer 118 is inert (e.g., a platinum or gold layer/material) in that it does not participate in any redox reactions but it is redox-sensitive or redox-active in the sense that it serves as both a source and sink of electrons (responding to the redox state of the contained sample 113). The active electrode layer 118 is considered inert because the process of transferring electrons does not change the material of the electrode or its oxidation state. The electrons are spontaneously transferred to the active electrode layer 118 from the contained sample 113 and from the active electrode layer 118 to the contained sample 113. A higher concentration of oxidized molecules (positive ORP value) implies a higher tendency for these molecules to accept electrons from the active electrode layer 118, whereas a higher concentration of reduced molecules (negative ORP value) implies a higher tendency for the molecules to give up electrons to the active electrode layer 118. Therefore, one either ends up with a loss of electrons on the active electrode layer 118, which results in a positive ORP value or one ends up with an excess of electrons on the active electrode layer 118, which results in a negative ORP value.

Different redox-active species are generated during bacterial metabolism and growth. That is, bacterial growth and/or metabolic processes involve the conversion of oxidized molecules into reduced ones. As the amount of bacteria in the contained sample 113 increases, the concentration of reduced molecules/compounds increases. This, in turn, causes the ORP of the contained sample 113 to decrease.

As a more specific example, the amount of electron donors from Table 1 below (e.g., the amount of energy carriers such as nicotinamide adenine dinucleotide (NADH) and flavin adenine dinucleotide ($FADH_2$)) in the contained sample 113 can change due to the growth of bacteria in the contained sample 113.

**TABLE 1:** Below is a "redox tower" visualizing potential electron donors and acceptors which can be utilized by bacteria during the course of metabolism. An electron donor will have a greater negative potential than the electron acceptor. In aerobic respiration for example, $O_2$ can serve as a terminal electron acceptor whereas in anaerobic respiration, the terminal electron acceptor can comprise $NO_3^-$, $Fe^{3+}$, $Mn^{4+}$, $SO_4^{2-}$, or $CO_2$.

| Electron Donor and Acceptor Pairs | Measured Standard Reduction Potential $E'_0$ (mV) | Standard Reduction Potential $E'_0$ (mV) range |
|---|---|---|
| Glucose $\rightleftharpoons$ 2 Pyruvate + $2e^-$ | -720 | -700 |

(continued)

| Electron Donor and Acceptor Pairs | Measured Standard Reduction Potential $E'_0$ (mV) | Standard Reduction Potential $E'_0$ (mV) range |
|---|---|---|
| Glucose $\leftrightharpoons$ 6 $CO_2$ + $24e^-$ | -500 | -500 |
| $H_2$ $\leftrightharpoons$ $2H^+$ + $2e^-$ | -420 | -400 |
| NADH $\leftrightharpoons$ $NAD^+$ + $2e^-$ | -320 | -300 |
| 2 GSH $\leftrightharpoons$ GSSG + $2e^-$<br>$H_2S$ $\leftrightharpoons$ $SO_4^{2-}$ + $8e^-$<br>$FADH_2$ $\leftrightharpoons$ FAD + $2H^+$ + $2e^-$ | -240<br>-220<br>-220 | -200 |
| Lactate $\leftrightharpoons$ Pyruvate + $2e^-$ | -190 | -100 |
| Succinate $\leftrightharpoons$ Fumarate + $2e^-$<br>Cyt b (red) $\leftrightharpoons$ Cyt b (ox) + $e^-$ | 33<br>80 | 0 |
| Ubiquinol $\leftrightharpoons$ Ubiquinone + $2e^-$ | 110 | 100 |
| Cyt c (red) $\leftrightharpoons$ Cyt c (ox) + $e^-$<br>Cyt a (red) $\leftrightharpoons$ Cyt a (ox) + $e^-$ | 250<br>290 | 200 |
| $NO_2^-$ + $H_2O$ $\leftrightharpoons$ $NO_3^-$ + $2e^-$ | 420 | 400 |
| $NH_4^+$ + $H_2O$ $\leftrightharpoons$ $NO_2^-$ + $6e^-$<br>$Mn^{2+}$ + $H_2O$ $\leftrightharpoons$ $MnO_2$ + $2e^-$ | 440<br>460 | |
| ½ $N_2$ + $3H_2O$ $\leftrightharpoons$ $NO_3^-$ + $5e^-$<br>$Fe^{2+}$ $\leftrightharpoons$ $Fe^{3+}$ + $1e^-$ | 740<br>770 | 700 |
| $H_2O$ $\leftrightharpoons$ ½ $O_2$ + $2H^+$ + $2e^-$ | 820 | 800 |

The reference electrode material 132 is chosen so that it maintains a constant potential through the measurement/monitoring and is not affected by or take part in any of the redox changes occurring in the contained sample 113.

The potential difference measured between the active electrode layer 118 and the reference electrode material 132 is measured in an open-circuit configuration. That is, no current flows through the system and the potential difference is measured using a very high impedance voltage measurement circuit or high impedance voltmeter integrated into the reader 200 (e.g., integrated into the signal readout control unit 210).

The ORP of the contained sample 113 is measured without any added reporter molecules or added redox mediators.

Fig. 3 illustrates one embodiment of a method 300 of preparing an output sample of bacteria 302 of a desired or target concentration 308 (or within acceptable error margins thereof).

The bacteria 302 can be of a genera selected from the group consisting of: *Acinetobacter, Acetobacter, Actinomyces, Aerococcus, Aeromonas, Agrobacterium, Anaplasma, Azorhizobium, Azotobacter, Bacillus, Bacteriodes, Bartonella, Bordetella, Borrelia, Brucella, Burkholderia, Calymmatobacterium, Campylobacter, Chlamydia, Chlamydophila, Citrobacter, Clostridium, Corynebacterium, Coxiella, Ehrlichia, Enterobacter, Enterococcus, Escherichia, Francisella, Fusobacterium, Gardnerella, Haemophilus, Helicobacter, Klebsiella, Lactobacillus, Legionella, Listeria, Methanobacterium, Microbacterium, Micrococcus, Morganella, Moraxella, Mycobacterium, Mycoplasma, Neisseria, Pandoraea, Pasteurella, Peptostreptococcus, Porphyromonas, Prevotella, Proteus, Providencia, Pseudomonas, Ralstonia, Raoultella, Rhizobium, Rickettsia, Rochalimaea, Rothia, Salmonella, Serratia, Shewanella, Shigella, Spirillum, Staphylococcus, Strenotrophomonas, Streptococcus, Streptomyces, Treponema, Vibrio, Wolbachia,* and *Yersinia.*

More specifically, the bacteria 302 can be of a species selected from the group consisting of: *Acinetobacter baumannii, Actinobacillus spp., Actinomycetes, Actinomyces spp.* (including but not limited to *Actinomyces israelii* and *Actinomyces naeslundii*), *Aeromonas spp.* (including but not limited to *Aeromonas hydrophila, Aeromonas veronii biovar*

*sobria (Aeromonas sobria),* and *Aeromonas caviae), Anaplasma phagocytophilum, Alcaligenes xylosoxidans, Actinobacillus actinomycetemcomitans, Bacillus spp.* (including but not limited to *Bacillus anthracis, Bacillus cereus, Bacillus subtilis, Bacillus thuringiensis,* and *Bacillus stearothermophilus*), *Bacteroides spp.* (including but not limited to *Bacteroides fragilis*), *Bartonella spp.* (including but not limited to *Bartonella bacilliformis* and *Bartonella henselae, Bifidobacterium spp., Bordetella spp.* (including but not limited to *Bordetella pertussis, Bordetella parapertussis,* and *Bordetella bronchiseptica), Borrelia spp.* (including but not limited to *Borrelia recurrentis,* and *Borrelia burgdorferi*), *Brucella spp.* (including but not limited to *Brucella abortus, Brucella canis, Brucella melintensis* and *Brucella suis*), *Burkholderia spp.* (including but not limited to *Burkholderia pseudomallei* and *Burkholderia cepacia*), *Campylobacter spp.* (including but not limited to *Campylobacter jejuni, Campylobacter coli, Campylobacter lari* and *Campylobacter fetus*), *Capnocytophaga spp., Cardiobacterium hominis, Chlamydia trachomatis, Chlamydophila pneumoniae, Chlamydophila psittaci, Citrobacter spp., Coxiella burnetii, Corynebacterium spp.* (including but not limited to, *Corynebacterium diphtheriae, Corynebacterium jeikeum* and *Corynebacterium*), *Clostridium spp.* (including but not limited to *Clostridium perfringens, Clostridium difficile, Clostridium botulinum* and *Clostridium tetani*), *Eikenella corrodens, Enterobacter spp.* (including but not limited to *Enterobacter aerogenes, Enterobacter agglomerans, Enterobacter cloacae and Escherichia coli,* including opportunistic *Escherichia coli,* including but not limited to enterotoxigenic *E. coli,* enteroinvasive *E. coli,* enteropathogenic *E. coli,* enterohemorrhagic *E. coli,* enteroaggregative *E. coli and* uropathogenic *E. coli*), *Enterococcus spp.* (including but not limited to *Enterococcus faecalis* and *Enterococcus faecium*), *Ehrlichia spp.* (including but not limited to *Ehrlichia chafeensia* and *Ehrlichia canis*), *Erysipelothrix rhusiopathiae, Eubacterium spp., Francisella tularensis, Fusobacterium nucleatum, Gardnerella vaginalis, Gemella morbillorum, Haemophilus spp.* (including but not limited to *Haemophilus influenzae, Haemophilus ducreyi, Haemophilus aegyptius, Haemophilus parainfluenzae, Haemophilus haemolyticus* and *Haemophilus parahaemolyticus, Helicobacter spp.* (including but not limited to *Helicobacter pylori, Helicobacter cinaedi* and *Helicobacter fennelliae*), *Kingella kingji, Klebsiella spp.* (including but not limited to *Klebsiella pneumoniae, Klebsiella granulomatis and Klebsiella oxytoca*), *Lactobacillus spp., Listeria monocytogenes, Leptospira interrogans, Legionella pneumophila, Leptospira interrogans, Peptostreptococcus spp., Moraxella catarrhalis, Morganella spp., Mobiluncus spp., Micrococcus spp., Mycobacterium spp.* (including but not limited to *Mycobacterium leprae, Mycobacterium tuberculosis, Mycobacterium intracellulare, Mycobacterium avium, Mycobacterium bovis,* and *Mycobacterium marinum*), *Mycoplasm spp.* (including but not limited to *Mycoplasma pneumoniae, Mycoplasma hominis,* and *Mycoplasma genitalium*), *Nocardia spp.* (including but not limited to *Nocardia asteroides, Nocardia cyriacigeorgica* and *Nocardia brasiliensis*), *Neisseria spp.* (including but not limited to *Neisseria gonorrhoeae* and *Neisseria meningitidis*), *Pasteurella multocida, Plesiomonas shigelloides, Prevotella spp., Porphyromonas spp., Prevotella melaninogenica, Proteus spp.* (including but not limited to *Proteus vulgaris* and *Proteus mirabilis*), *Providencia spp.* (including but not limited to *Providencia alcalifaciens, Providencia rettgeri* and *Providencia stuartii*), *Pseudomonas aeruginosa, Propionibacterium acnes, Rhodococcus equi, Rickettsia spp.* (including but not limited to *Rickettsia rickettsii, Rickettsia akari* and *Rickettsia prowazekii, Orientia tsutsugamushi* (formerly: *Rickettsia tsutsugamushi*) and *Rickettsia typhi*), *Rhodococcus spp., Stenotrophomonas maltophilia, Salmonella spp.* (including but not limited to *Salmonella enterica, Salmonella typhi, Salmonella paratyphi, Salmonella enteritidis, Salmonella cholerasuis and Salmonella typhimurium*), *Serratia spp.* (including but not limited to *Serratia marcesans and Serratia liquifaciens*), *Shigella spp.* (including but not limited to *Shigella dysenteriae, Shigella flexneri, Shigella boydii* and *Shigella sonnei*), *Staphylococcus spp.* (including but not limited to *Staphylococcus aureus, Staphylococcus epidermidis, Staphylococcus hemolyticus, Staphylococcus saprophyticus*), *Streptococcus spp.* (including but not limited to *Streptococcus pneumoniae* (for example chloramphenicol-resistant serotype 4 *Streptococcus pneumoniae,* spectinomycin-resistant serotype 6B *Streptococcus pneumoniae,* streptomycin-resistant serotype 9V *Streptococcus pneumoniae,* erythromycin-resistant serotype 14 *Streptococcus pneumoniae,* optochin-resistant serotype 14 *Streptococcus pneumoniae,* rifampicin-resistant serotype 18C *Streptococcus pneumoniae,* tetracycline-resistant serotype 19F *Streptococcus pneumoniae,* penicillin-resistant serotype 19F *Streptococcus pneumoniae,* and trimethoprim-resistant serotype 23F *Streptococcus pneumoniae,* chloramphenicol-resistant serotype 4 *Streptococcus pneumoniae,* spectinomycin-resistant serotype 6B *Streptococcus pneumoniae,* streptomycin-resistant serotype 9V *Streptococcus pneumoniae,* optochin-resistant serotype 14 *Streptococcus pneumoniae,* rifampicin-resistant serotype 18C *Streptococcus pneumoniae,* penicillin-resistant serotype 19F *Streptococcus pneumoniae,* or trimethoprim-resistant serotype 23F *Streptococcus pneumoniae*), *Streptococcus agalactiae, Streptococcus mutans, Streptococcus pyogenes,* Group A *Streptococci, Streptococcus pyogenes,* Group B *Streptococci, Streptococcus agalactiae,* Group C *Streptococci, Streptococcus anginosus, Streptococcus equismilis,* Group D *Streptococci, Streptococcus bovis,* Group F *Streptococci, Streptococcus anginosus,* and Group G *Streptococci*), *Spirillum minus, Streptobacillus moniliformi, Treponema spp.* (including but not limited to *Treponema carateum, Treponema petenue, Treponema pallidum* and *Treponema endemicum, Tropheryma whippelii, Ureaplasma urealyticum, Veillonella spp., Vibrio spp.* (including but not limited to *Vibrio cholerae, Vibrio parahemolyticus, Vibrio vulnificus, Vibrio parahaemolyticus, Vibrio vulnificus, Vibrio alginolyticus, Vibrio mimicus, Vibrio hollisae, Vibrio fluvialis, Vibrio metchnikovii, Vibrio damsela* and *Vibrio furnisii*), *Xanthomonas maltophilia,* and *Yersinia spp.* (including but not limited to Yersinia enterocolitica, Yersinia pestis, and *Yersinia pseudotuberculosis*).

The method 300 can be used to prepare an output sample of bacteria 302 of a desired or target concentration (or within acceptable error margins thereof) when the bacteria 302 is an obligate or strict aerobe. The method 300 can also be used to prepare an output sample of bacteria 302 of a desired or target concentration (or within acceptable error margins thereof) when the bacteria 302 is a facultative anaerobe. The method 300 can further be used to prepare an output sample of bacteria 302 of a desired or target concentration (or within acceptable error margins thereof) when the bacteria 302 is a gram-negative bacteria.

One unexpected discovery made by the applicants is that the method and system disclosed herein works especially well to prepare an output sample of a desired or target concentration (or within acceptable error margins thereof) from a source sample comprising bacteria that are classified or considered as obligate or strict aerobes. For example, for certain species of obligate or strict aerobes, such as *Acinetobacter baumannii* (ABa) and *Pseudomonas aeruginosa* (PAe), the methods disclosed herein have been shown to significantly decrease sample preparation times for such obligate or strict aerobes compared to methods that do not use aeration.

Another unexpected discovery made by the applicants is that the method and system disclosed herein also works well to prepare an output sample of a desired or target concentration (or within acceptable error margins thereof) from a source sample comprising bacteria that are classified or considered as facultative anaerobes such as *Escherichia coli* (ECo), *Serratia marcescens* (SMa), and *Proteus mirabilis* (PMi). For example, for certain species of facultative anaerobes, the methods disclosed herein have been shown to decrease sample preparation times for such facultative anaerobes compared to methods that do not use aeration.

Yet another unexpected discovery made by the applicants is that the method and system disclosed herein works well to prepare an output sample of a desired or target concentration (or within acceptable error margins thereof) from a source sample comprising gram-negative bacteria that are classified or considered as facultative anaerobes or obligate/strict aerobes. For example, the applicants discovered that the method disclosed herein works especially well for preparing an output sample of a desired or target concentration, or within acceptable error margins thereof, from a source sample comprising the following species of gram-negative bacteria: ECo, SMa, PMi, *Proteous vulgaris* (PVu), ABa, PAe, *Klebsiella pneumoniae* (KPn), *Enterobacter cloacae* (ECl), *Klebsiella oxytoca* (KOx), *Klebsiella aerogenes* (KAe), *Citrobacter braakii* (CBr), *Citrobacter freundii* (CFr), and *Citrobacter koseri* (CKo).

In alternative embodiments, the method 300, devices, and system 301 disclosed herein can also be used to prepare an output sample of mold or fungi of a desired or target concentration (or within acceptable error margins thereof). The fungi can be of a genera selected from the group consisting of: *Candida* and *Cryptococcus.* More specifically, the fungi can be of a species selected from the group consisting of: *Candida spp.* (including but not limited to *Candida albicans, Candida glabrata, Candida tropicalis, Candida parapsilosis,* and *Candida krusei*)*, Aspergillus spp.* (including but not limited to *Aspergillus fumigatous, Aspergillus flavus, Aspergillus clavatus*)*, Cryptococcous spp.* (including but not limited to *Cryptococcus neoformans, Cryptococcus gattii, Cryptococcus laurentii,* and *Cryptococcus albidus*)*, Fusarium spp.* (including but not limited to *Fusarium oxysporum, Fusarium solani, Fusarium verticillioides,* and *Fusarium proliferatum*)*, Rhizopus oryzae, Penicillium marneffei, Coccidiodes immitis,* and *Blastomyces dermatitidis.*

The method 300 can comprise diluting an aliquot of a source sample comprising bacteria 302 by a dilution factor to yield a diluted sample 304 in step 300A. In certain embodiments, the source sample can comprise a bodily fluid or a bacterial culture derived therefrom. The bodily fluid can be at least one of blood, urine, serum, plasma, saliva, sputum, semen, breast milk, joint fluid, spinal fluid such as cerebrospinal fluid, wound material, mucus, fluid accompanying stool, vaginal secretions, synovial fluid, pleural fluid, peritoneal fluid, pericardial fluid, and amniotic fluid. For example, the source sample be a bacterial culture or a re-suspended bacterial culture derived from a bodily fluid (or swab). More specifically, the source sample can be a positive blood culture (PBC).

In additional embodiments not shown in Fig. 3, the source sample can be filtered before step 300A. This filtering step can involve filtering the source sample using a laboratory filter, a benchtop filter, a medical filter, a microfluidic filter, a syringe filter, a blood filter, a urine filter, or a combination thereof to filter out debris, inorganic material, and larger cellular components including blood cells or epithelial cells from the source sample.

The aliquot of the source sample can be diluted using a dilutive solution 306. The dilutive solution 306 can comprise growth media (e.g., bacteria growth media) or a growth inducer. In some embodiment, the dilutive solution 306 can be a solution containing a cation-adjusted Mueller Hinton Broth (CAMHB), a glucose supplemented Mueller Hinton broth (MHG), a CAMHB-LHB mix, bacto-tryptone, tryptic soy digest, yeast extract, beef extract, starch, acid hydrolysate of casein, calcium chloride, magnesium chloride, sodium chloride, blood or lysed blood including lysed horse blood (LHB), glucose or other carbohydrates, or a combination thereof. The growth inducer can comprise a carbon-based inducer, a nitrogen-based inducer, a mineral, a trace element, a biological growth factor, or any combination thereof. For example, the growth inducer can include but is not limited to a carbohydrate such as glucose or starches, ammonia, magnesium, amino acids, casamino acids, vitamins, peptides, blood, or a combination thereof. In one example embodiment, the dilutive solution 306 can comprise tryptone, yeast extract, sodium chloride, starch, water, and glucose.

The dilution factor can be between about 1:1 to 1:100 or 1:1 to 1: 1000. In some embodiments, the dilution factor can be between about 1:10 to 1:100. More specifically, the dilution factor can be between about 1:10 and 1:50. For example, the dilution factor can be about 1:30 when the source sample is a positive blood culture. As a more specific

example, a 30 μL source sample can be diluted into 1 mL of the dilutive solution 306.

Although Fig. 3 illustrates only one aliquot of the source sample being diluted in step 300A, it is contemplated by this disclosure that additional aliquots of the source sample can be diluted to the same dilution ratio or different dilution ratios to yield additional diluted samples 304 (e.g., a second diluted sample, a third diluted sample, a fourth diluted sample, etc.). The additional diluted samples 304 can be used to generate internal controls or redundant samples.

The method 300 can also comprise introducing an aliquot of the diluted sample 304 comprising the bacteria 302 into a container chamber 102 of the sensor apparatus 100 in step 300B. The amount of diluted sample 304 introduced can depend on a volume of the chamber cavity 112 of the container chamber 102 (see, e.g., Fig. 1B). For example, a 1 mL aliquot of the diluted sample 304 can be introduced into the container chamber 102 of the sensor apparatus 100.

The aliquot of the diluted sample 304 within the container chamber 102 can be in fluid communication with both an active sensor 106 and a reference sensor 108 of the sensor apparatus 100. For purposes of this disclosure, the aliquot of the diluted sample 304 within the container chamber 102 will be referred to as the contained sample 113.

As previously discussed, the active sensor 106 can be coupled to at least part of a chamber lateral wall 110 of the container chamber 102. The active sensor 106 can also comprise an active electrode material or active electrode layer 118 that faces the chamber cavity 112 such that the contained sample 113 is in fluid contact with the active electrode material or active electrode layer 118 when the contained sample 113 fills the chamber cavity 112 of the container chamber 102.

Also, as previously discussed, the reference sensor 108 can comprise a reference electrode material 132 and a wick or wicking component 134 in fluid communication with the chamber cavity 112 of the container chamber 102. When the container chamber 102 is filled with the contained sample 113, at least some of the contained sample 113 in the container chamber 102 can be drawn up, absorbed, or otherwise wicked by at least a portion of the wicking component 134 in a direction of the wick proximal end 140. Since the reference electrode material 132 is disposed at the wick proximal end 140 (see, e.g., Fig. 1D), the contained sample 113 can be in fluid contact with the reference electrode material 132 via the wicking component 134.

The method 300 can further comprising placing the assembled sensor apparatus 100 (the assembled sensor apparatus 100 is when the container cap 104 is fastened to the sample-filled container chamber 102) into a container receiving space of the reader 200 in step 300C. For example, a user can lift the lid 206 of the reader 200 to insert the assembled sensor apparatus 100 into the container receiving space of the reader 200.

As previously discussed, when the sensor apparatus 100 is positioned within the container receiving space, a reference electrode contact 216 of the reader 200 (see, e.g., Fig. 2C) can be placed or moved into electrical contact with the reference electrode material 132 positioned on the container cap 104 (see, e.g., Fig. 1D) of the sensor apparatus 100. Moreover, when the sensor apparatus 100 is positioned within the container receiving space, an active electrode contact 218 of the reader 200 can be placed or moved into electrical contact with a conductive layer 160 (see, e.g., Figs. 1C, 1D, and 2C) or conductive contact of the active sensor 106. In this manner, both the active sensor 106 and the reference sensor 108 can be electrically coupled to the reader 200.

The reference electrode contact 216 and the active electrode contact 218 can be electrically coupled to a signal readout control unit 210 (see, e.g., Fig. 2B). The signal readout control unit 210 can comprise one or more processors, chipsets, or chip modules programmed to convert and read signals obtained from the active sensor 106 and the reference sensor 108 of the sensor apparatus 100. For example, the signal readout control unit 210 can determine an ORP of the contained sample 113 within the sensor apparatus 100 based on a potential difference measured between the active electrode layer 118 and the reference electrode material 132.

At this point, a user (e.g., a laboratory technician or clinician) of the reader 200 can input a desired or target concentration 308 to the reader 200. For example, the user can apply certain touch inputs to the display 204 of the reader 200 to select a preset bacteria concentration level or input the desired or target concentration 308. Also, for example, the user can input the desired or target concentration 308 via a keyboard or other type of input device communicatively coupled to the reader 200. Alternatively, the user can also input the desired or target concentration 308 into a computing device 310 (e.g., a tablet or laptop) communicatively coupled to the reader 200. The computing device 310 can transmit the desired or target concentration 308 to the reader 200 via a wireless communicate protocol or a wired connection.

The desired or target concentration 308 can be a bacterial concentration level required as part of a downstream testing protocol such as an antimicrobial or antibiotic susceptibility test (AST). In certain embodiments, the desired or target concentration 308 can be expressed or displayed as colony forming units (CFUs) per mL. In other embodiments, the desired or target concentration 308 can be expressed or displayed in terms of McFarland standards (e.g., 0.5 McFarland, 1.0 McFarland, 2.0 McFarland, etc.).

In some embodiments, the desired or target concentration 308 can be between about $1.4 \times 10^8$ CFU/mL and $1.6 \times 10^8$ CFU/mL. For example, the desired or target concentration 308 can be about $1.5 \times 10^8$ CFU/mL (also referred to as a 0.5 McFarland standard). In other embodiments, the desired or target concentration 308 can be greater than $1.6 \times 10^8$ CFU/mL or less than $1.4 \times 10^8$ CFU/mL.

In some embodiments, the user can also input certain information concerning a classification (e.g., a genus, family, or order) of the bacteria 302 or a characteristic of the bacteria 302. For example, the user can perform a Gram-stain test of the bacteria 302 prior to introducing the diluted sample 304 into the sensor apparatus 100. The user can then input into the reader 200 whether the bacteria 302 is gram-positive or gram-negative based on the Gram-stain test. In some cases, the reader 200 can retrieve one or more look-up tables (LUTs) tailored to the classification or characteristic of the bacteria 302 provided by the user.

The method 300 can also comprise incubating and aerating the contained sample 113 in step 300D. In some embodiments, the contained sample 113 within the sensor apparatus 100 can be incubated and aerated simultaneously. In other embodiments, the contained sample 113 within the sensor apparatus 100 can begin the incubation period without aeration initially or begin the aeration period without incubation initially. In all such embodiments, there can be a period of time where the contained sample 113 within the sensor apparatus 100 is both incubated and aerated.

The contained sample 113 within the sensor apparatus 100 can be incubated at an incubation temperature. In some embodiments, the incubation temperature can be between about 30 °C and 40 °C (e.g., about 35 °C $\pm$ 2 °C). In other embodiments, the incubation temperature can be between about 25 °C and 30°C. As previously discussed, the sensor apparatus 100 comprising the contained sample 113 can be incubated while housed within the reader 200. For example, the thermal control module 212 of the reader 200 can control the incubation of the sample-filled sensor apparatus 100. The reader 200 can incubate the sensor apparatus 100 by heating at least part of the sensor apparatus 100 via the heating block 220 (see, e.g., Fig. 2C). In some embodiments, the heating block 220 can heat a lateral of the container chamber 102 opposite the active sensor 106. In certain embodiments, the heating block 220 can heat part of the bottom or base of the container chamber 102 or partially surround or cradle the container chamber 102 to heat the sensor apparatus 100.

The contained sample 113 within the sensor apparatus 100 can be aerated at an aeration flow rate or gas dispense rate. The aeration flow rate can be between 7.0 microliter ($\mu$L) per second per milliliter (mL) of the contained sample 113 and 10.0 $\mu$L per second per mL of the contained sample 113. More specifically, the contained sample 113 within the sensor apparatus 100 can be aerated at a flow rate of about 8.8 ($\pm$0.9) $\mu$L per second per mL of the contained sample 113. As previously discussed, the contained sample 113 can be aerated using a motorized piston pump. The motorized piston pump can be housed or contained within the reader 200. Aeration of the contained sample 113 can be controlled by the aeration control module 214 of the reader 200.

In some embodiments, the contained sample 113 within the sensor apparatus 100 can be aerated in accordance with an aeration cycle. The aeration cycle can include an aeration period followed by a non-aerated period where no gas or ambient air is pumped into the container chamber 102 (see, e.g., Fig. 2B).

In certain embodiments, the aeration period can be longer than the non-aerated period. For example, the aeration period can be between about 7 minutes and 10 minutes and the non-aerated period can be between about 3 seconds and 10 seconds. As a more specific example, the contained sample 113 within the container chamber 102 can be aerated repeatedly at a flow rate or dispense rate of about 10.0 $\mu$L per second per mL of the contained sample 113 for a period about 8 minutes followed by a non-aerated period of about 5 seconds.

The method 300 can also comprise monitoring a change in the ORP of the contained sample 113 within the sensor apparatus 100 in step 300E. The ORP of the contained sample 113 can be monitored or measured as soon as the sensor apparatus 100 is positioned within the reader 200 and the user has inputted the desired or target concentration 308. The ORP of the contained sample 113 can be monitored or measured during the pendency of the incubation period and/or aeration period.

As previously discussed, in some embodiments, the signal readout control unit 210 of the reader 200 can monitor the ORP of the contained sample 113 within the container chamber 102 of the sensor apparatus 100. For example, as part of the ORP monitoring process, the ORP of the contained sample 113 can be sampled or determined multiple times per second and such ORP values can be recorded in conjunction with an elapsed time 313. The reader 200 can display the change in ORP as a function of the elapsed time 313 as an ORP growth curve 311. As a more specific example, the ORP growth curve 311 can be rendered and shown to the user via the display 204 of the reader 200 or via a display of a computing device 310 communicatively coupled to the reader 200. As the amount of bacteria in the contained sample 113 increases (the bacterial concentration increases), the amount of reduced molecules/compounds in the contained sample 113 also increases. This, in turn, causes the ORP of the contained sample 113 to decrease or the ORP value to become more negative.

The method 300 can further comprise retrieving a species-agnostic look-up table (LUT) 312 from a database in step 300F. The species-agnostic LUT 312 can be retrieved in response to the user inputting the desired or target concentration 308. In other embodiments, the species-agnostic LUT 312 can be retrieved once the ORP of the contained sample 113 is being monitored by the reader 200. For example, one or more processors of the reader 200 can be programmed to retrieve the species-agnostic LUT 312 from a memory or storage unit of the reader 200. In other embodiments, the one or more processors of the reader 200 can be programmed to retrieve the species-agnostic LUT 312 from a database stored on the computing device 310 or a database in the cloud.

The species-agnostic LUT 312 can comprise a plurality of species-agnostic ORP change amounts 314 and species-agnostic bacterial concentrations 316. Each species-agnostic bacterial concentration 316 can have a species-agnostic ORP change amount 314 associated with the species-agnostic bacterial concentration 316.

The species-agnostic LUT 312 can be constructed or generated from a plurality of species-specific LUTs 406 and/or strain-specific LUTs 404 (see, e.g., Fig. 4). For example, each of the species-agnostic ORP change amounts 314 or each of the species-agnostic bacterial concentrations 316 can be averaged from multiple ORP change amounts or bacterial concentrations across multiple LUTs, respectively. Constructing or generating the species-agnostic LUT 312 from species-specific LUTs 406 and/or strain-specific LUTs 404 will be discussed in more detail in later sections.

The method 300 can further comprise determining whether the desired or target concentration 308 is included in the species-agnostic LUT 312 in step 300G. For example, the one or more processors of the reader 200 can query the bacterial concentration field (i.e., the species-agnostic bacterial concentrations 316) in the species-agnostic LUT 312 using the desired or target concentration 308 received from the user. If the one or more processors of the reader 200 determines that the desired or target concentration 308 is included in the species-agnostic LUT 312, the one or more processors of the reader 200 can select one of the species-agnostic ORP change amounts 314 as a threshold ORP change amount 318 when the species-agnostic ORP change amount 314 selected is associated with one of the species-agnostic bacterial concentrations 316 equal or substantially equal to the desired or target concentration 308 in step 300H. In this manner, the reader 200 relies primarily on the species-agnostic LUT 312 to set the threshold ORP change amount 318.

However, if the one or more processors of the reader 200 determines that the desired or target concentration 308 is not included in the species-agnostic LUT 312, the one or more processors of the reader 200 can calculate a time-to-target concentration ($t_{target}$) 320 in step 300I. Calculating the time-to-target concentration 320 will be discussed in more detail in later sections.

In certain embodiments, the one or more processors of the reader 200 can opt to calculate the time-to-target concentration 320 even if the desired or target concentration 308 is included in the species-agnostic LUT 312. For example, the one or more processors of the reader 200 can opt for this calculation based on certain heuristics or preset rules that dictate when the threshold ORP change amount 318 from the species-agnostic LUT 312 is considered too high/too large or may be prone to error. In this case, the one or more processors of the reader 200 can make the determination to calculate the time-to-target concentration 320 rather than rely on certain ORP values (i.e., species-agnostic ORP change amounts 314) from the species-agnostic LUT 312. For example, the one or more processors of the reader 200 can decide that certain smaller species-agnostic ORP change amounts 314 are more accurate or less prone to error based on real-time or near-real-time analysis of the behavior of the ORP growth curve 311 (for example, if the ORP signal monitored is beginning to flatten out). In this case, the one or more processors of the reader 200 can determine that certain smaller species-agnostic ORP change amounts 314 from the species-agnostic LUT 312 are more applicable or less prone to error and use such ORP change amounts in calculating the time-to-target concentration 320.

The method 300 can also comprise determining that the bacteria in the contained sample 113 has reached the desired or target concentration 308 (or within acceptable error margins thereof) in step 300J. For example, the one or more processors of the reader 200 can determine that the bacteria in the contained sample 113 has reached the desired or target concentration 308 (or within acceptable error margins thereof) when either the change in the ORP of the contained sample 113 monitored in real-time or near-real-time by the reader 200 has reached the threshold ORP change amount 318 (or within acceptable error margins thereof) (see, also, step 300H) or when the elapsed time 313 has reached the calculated time-to-target concentration 320 (see, also, step 300I).

The method 300 can further comprise cooling the contained sample 113 within the sensor apparatus 100 when the concentration of the bacteria in the contained sample 113 is determined to have reached the desired or target concentration 308 (or within acceptable error margins thereof) in step 300K. The contained sample 113 can be cooled at a cooling temperature between about 4 °C and 25 °C. The sensor apparatus 100 can be cooled within the reader 200. For example, the thermal control module 212 can also be used to cool the contained sample 113 to between about 4 °C and 25 °C. Cooling the contained sample 113 is needed to prevent the bacteria within the contained sample 113 from continuing to grow or the bacterial concentration from increasing any further.

Step 300K can also comprise the reader 200 alerting the user that the bacteria in the contained sample 113 has reached the desired or target concentration 308 (or within acceptable error margins thereof) and the output sample is now ready for downstream testing. For example, the reader 200 can comprise a speaker and the speaker can generate an audible alert or sound an alarm to notify the user that the bacteria in the contained sample 113 has reached the desired or target concentration 308 (or within acceptable error margins thereof). In additional embodiments, the reader 200 can render a visual or graphic alert via its display 204 informing the user that the bacteria in the contained sample 113 has reached the desired or target concentration 308 (or within acceptable error margins thereof) and the output sample is now ready for downstream testing.

As shown in Fig. 3, a laboratory technician or clinician can use the method 300 to prepare an output sample of a desired or target concentration 308 (or within acceptable error margins thereof) without any prior knowledge of a species of the bacteria in the contained sample 113 or having to ascertain the species of the bacteria in the contained sample 113. This can significantly reduce sample preparation times or reduce the amount of human effort required to prepare an output sample since the laboratory technician or clinician no longer has to subject the source sample or contained sample 113 to a separate species-identification protocol.

The method steps depicted in Fig. 3 do not require the particular order shown to achieve the desired result. Moreover, certain steps or processes may be omitted or occur in parallel in order to achieve the desired result. In addition, other devices or apparatus can be used in lieu of the devices or apparatus shown in of Fig. 3.

Fig. 4 illustrates that the species-agnostic LUT 312 can be generated from multiple constituent LUTs 402. In some embodiments, the species-agnostic LUT 312 can be generated from at least three constituent LUTs 402. For example, the species-agnostic LUT 312 can be generated from between five and eight constituent LUTs 402. In other embodiments, the species-agnostic LUT 312 can be generated from nine or more constituent LUTs 402.

Each of the constituent LUTs 402 can be either a strain-specific LUT 404 or a species-specific LUT 406. A species-specific LUT 406 can be generated from multiple strain-specific LUTs 404 comprising bacteria of the same species. Each of the strain-specific LUTs 404 can be compiled using ORP measurements and bacterial concentration measurements taken concurrently of a reference bacterial sample 408.

In some embodiments, the species-agnostic LUT 312 can be generated from multiple (at least three) strain-specific LUTs 404. In other embodiments, the species-agnostic LUT 312 can be generated from multiple (at least three) species-specific LUTs 406. In additional embodiments, the species-agnostic LUT 312 can be generated from a mixture of strain-specific LUTs 404 and species-specific LUTs 406.

For example, the at least three constituent LUTs 402 can include a first LUT, a second LUT, and a third LUT. Each of the first LUT, the second LUT, or the third LUT can be either a strain-specific LUT 404 or a species-specific LUT 406. The first LUT, the second LUT, and the third LUT can be generated using concurrent ORP and bacterial concentration measurements made or taken of a first reference bacterial sample, a second reference bacterial sample, and a third reference bacterial sample, respectively. The first reference bacterial sample can comprise a bacteria of a first species, the second reference bacterial sample can comprise a bacteria of a second species different from the first species, and the third reference bacterial sample can comprise a bacteria of a third species different from either the first species or the second species.

Each of the constituent LUTs 402 can comprise constituent LUT ORP change amounts 410 and constituent LUT bacterial concentrations 412. In some embodiments, the constituent ORP change amounts 410 can be the same as the species-agnostic ORP change amounts 314. In these embodiments, the constituent LUT bacteria concentrations 412 associated with each of the constituent ORP change amounts 410 can be averaged across the multiple constituent LUTs 402 to obtain the species-agnostic bacterial concentrations 316.

For example, Fig. 5 illustrates a species-agnostic LUT 312 generated from six strain-specific LUTs 404. As a more specific example, the six strain-specific LUTs 404 can include LUTs representing the PSC-91 strain of ECo, the PSC-38 strain of KPn, the UCLA-126 strain of ABa, the PSC-30 strain of PAe, the UCLA-32 strain of PVu, and the CDC-91 strain of SMa. The strain-specific bacterial concentrations (or the various constituent LUT bacteria concentrations 412) across the six LUTs are averaged to obtain each of the species-agnostic bacterial concentrations 316 included as part of the species-agnostic LUT 312.

Although not shown in Fig. 5, it is contemplated by this disclosure that the species-agnostic LUT 312 can also be generated from multiple species-specific LUTs 406 or a mixture of species-specific LUTs 406 and strain-specific LUTs 404. For example, a species-specific LUT 406 can be generated for SMa from multiple strain-specific LUTs 404 of SMa including LUTs representing the CDC-27 strain of SMa, the CDC-91 strain of SMa, the CDC-99 strain of SMa, the CDC-121 strain of SMa, the CDC-122 strain of SMa, the CDC-130 strain of SMa, or a combination thereof. As another example, a species-specific LUT 406 can also be generated for *Staphylococcus aureus* (SAu) from multiple strain-specific LUTs 404 for SAu including LUTs comprising the wildtype strain of SAu, the CDC-483 strain of SAu, the CDC-475 strain of SAu, the ATCC43300 strain of SAu, or a combination thereof.

Referring back to Fig. 4, the method 400 of generating the species-agnostic LUT 312 can begin with preparing at least three reference bacterial samples 408. The at least three reference bacterial samples 408 can then be used to prepare the at least three constituent LUTs 402.

In some embodiments, between six and eight reference bacterial samples 408 can be prepared. In other embodiments, nine or more reference bacterial samples 408 can be prepared. The accuracy of the LUTs (including any of the species-specific LUTs 406 and the species-agnostic LUT 312) can be improved or enhanced when more reference bacterial samples 408 are used to generate such LUTs.

The reference bacterial samples 408 can be prepared by re-suspending plated colonies of bacteria of a known species and/or strain into liquid growth media such as the dilutive solution 306. An aliquot (e.g., 1 mL) of the re-suspended bacterial samples can then be introduced into an instance of the sensor apparatus 100. As shown in Fig. 4, each of the reference bacterial samples 408 can be introduced into its own sensor apparatus 100. The reference bacterial samples 408 can also be prepared such that each of the samples contain bacteria at the same initial concentration. For example, the initial concentration of bacteria in each of the reference bacterial samples 408 can be approximately 1 x $10^7$ (1e7) CFU/mL or 5 x $10^7$ (5e7) CFU/mL.

The ORP of each of the reference bacterial samples 408 can be monitored by a reader 200. For example, a sensor apparatus 100 containing the reference bacterial sample 408 can be placed within the container receiving space of the reader 200 and the reader 200 can be programmed to monitor changes in the ORP of the reference bacterial sample 408 over a period of time. Concurrent with this monitoring, the optical density (O.D.) of the reference bacterial sample 408 can also be measured at specific time intervals 414. For example, the specific time intervals 414 can be every several minutes such as every 15 minutes. In other embodiments, the specific time intervals 414 can be every 5 minutes, every 10 minutes, every 20 minutes, or every 30 minutes. For example, the ORP of the reference bacterial sample 408 can be monitored over a period of 180 minutes. Concurrent with this monitoring, the O.D. of this reference bacterial sample 408 can be periodically measured every 15 minutes over this 180-minute period.

The reader 200 can incubate and aerate the reference bacterial sample 408 similar to how it incubates and aerates the contained sample 113. The reader 200 can incubate the reference bacterial sample 408 at an incubation temperature of between about 30 °C and 40 °C (e.g., about 35 °C $\pm$ 2 °C). The reader 200 can also aerate the reference bacterial sample 408 between 7.0 $\mu$L per second per mL of the reference bacterial sample 408 and 10.0 $\mu$L per second per mL of the reference bacterial sample 408. More specifically, the reference bacterial sample 408 within the sensor apparatus 100 can be aerated at a flow rate of about 8.8 ($\pm$0.9) $\mu$L per second per mL of the reference bacterial sample 408.

In some embodiments, the reference bacterial sample 408 within the sensor apparatus 100 can be aerated in accordance with an aeration cycle. The aeration cycle can include an aeration period followed by a non-aerated period where no gas or ambient air is pumped into the container chamber 102. In certain embodiments, the aeration period can be longer than the non-aerated period. For example, the aeration period can be between about 7 minutes and 10 minutes and the non-aerated period can be between about 3 seconds and 10 seconds. As a more specific example, the reference bacterial sample 408 within the container chamber 102 of the sensor apparatus 100 can be aerated repeatedly at a flow rate or dispense rate of about 10.0 $\mu$L per second per mL of the reference bacterial sample 408 for a period about 8 minutes followed by a non-aerated period of about 5 seconds.

In some embodiments, O.D. measurements 417 can be conducted at a wavelength of 600 nm (OD600 measurements) using a spectrophotometry device 416 or system (e.g., UV-Vis spectrophotometry device). In certain embodiments, the sensor apparatus 100 can be removed from the reader 200 at the end of each of the specific time intervals 414 and the reference bacterial sample 408 can be transferred to another container or tube compatible with the spectrophotometry device 416 or system. In other embodiments, the sensor apparatus 100 can be designed or otherwise configured to work directly with certain types of spectrophotometry devices 416 or systems such that the O.D. of the reference bacterial sample 408 can be measured even when the reference bacterial sample 408 is within the container chamber 102 of the sensor apparatus 100.

In some embodiments, the spectrophotometry device 416 or system can be communicatively coupled to the computing device 310 which is, in turn, communicatively coupled to the reader(s) 200. The computing device 310 can record and store the results of the O.D. measurements 417 and the ORP monitoring in one or more databases stored in a memory of the computing device 310 or a cloud-based database accessible to the computing device 310.

In other embodiments, the spectrophotometry device 416 or system can be communicatively coupled directly to the reader 200 and the reader 200 can store the results of the O.D. measurements 417 along with the ORP change amounts.

The O.D. measurements 417 can be converted to reference sample bacterial concentrations 418 (expressed in CFU/mL) using a conversion factor. For example, one or more processors of the computing device 310 can be programmed to convert the results of the O.D. measurements 417 to reference sample bacterial concentrations 418 using the conversion factor. For example, the results of the O.D. measurements 417 can be converted to reference sample bacterial concentrations 418 by multiplying the results of the O.D. measurements 417 by a numerical conversion factor (e.g., O.D. x (1.76 x $10^9$)). The conversion factors are usually instrument dependent and vary from instrument to instrument.

In certain embodiments, a plate count assay or a flow cytometry assay can be conducted to determine the reference sample bacterial concentrations 418 in lieu of or in addition to the O.D. measurements 417.

The computing device 310 can then generate a strain-specific LUT 404 by associating each of the reference sample bacterial concentrations 418 (converted from an O.D. measurement 417) with a measured change in the ORP of the reference bacterial sample 408. For example, each of the reference sample bacterial concentrations 418 can be associated with a measured change in the ORP of the reference bacterial sample 408 as determined by the reader 200. Moreover, the reference sample bacterial concentrations 418 can then be included as the constituent LUT bacterial concentrations 412 for a particular strain-specific LUT 404 and the changes in the ORP of the reference bacterial sample 408 can be included as the constituent LUT ORP change amounts 410 of this particular strain-specific LUT 404.

This process can then be repeated for each of the other reference bacterial samples 408 until at least three strain-specific LUTs 404 are compiled. In some embodiments, numerous strain-specific LUTs 404 are created which are then used to create multiple species-specific LUTs 406. Such species-specific LUTs 406, or a combination of species-specific LUTs 406 and strain-specific LUTs 404, can then be used to create the species-agnostic LUT 312.

As previously discussed, the LUTs (including any of the species-agnostic LUT 312, the strain-specific LUTs 404, and the species-specific LUTs 406) can be stored as part of a database software program in a memory of the reader 200, the computing device 310, communicatively coupled to the reader 200, or a combination thereof. In other embodiments, the LUTs can be stored as part of a database software program in a computing cloud or a remote server accessible to the reader 200 and/or the computing device 310 over a network.

In some embodiments, multiple species-agnostic LUTs 312 can be prepared. In these embodiments, the species-agnostic LUTs 312 can be organized by genus, family, order, class, phylum, kingdom, or domain. Furthermore, certain species-agnostic LUTs 312 can also be organized by microbial characteristics, such as Gram-type, or functional capabilities, such as the ability to hydrolyze certain proteins or molecules, can also be selected or retrieved.

Fig. 6A and 6B illustrate results from 41 trial runs that were performed to evaluate the effectiveness of the method 300 and system 301 (the sensor apparatus 100 and reader 200) disclosed herein to prepare output samples of a desired or target concentration 308 (or within acceptable error margins thereof). All output samples were prepared with 1.5 x $10^8$ CFU/mL as the desired or target concentration 308. As shown in Fig. 6A, the 41 trial runs included source samples comprising ten different species of Gram-negative bacteria. These species included: PAe, ABa, ECo, KPn, ECl, KOx, PMi, KAe, SMa, and CFr. The species of bacteria within such source samples were determined to ensure that the method 300 performed equally well for different types of bacteria. It should be understood by one of ordinary skill in the art that the species of bacteria with a source sample does not need to be identified prior to using the method 300 to prepare an output sample.

In preparing the output samples, a threshold ORP change amount 318 ($\Delta ORP_{Threshold}$ = -60 mV) was selected from the species-agnostic LUT 312 shown in Fig. 5 based on the desired or target concentration 308 (1.5 x $10^8$ CFU/mL). The larger graph of Fig. 6A shows these results with final output sample concentrations plotted against the various bacterial species. The final output sample concentrations were determined using O.D. measurements and/or traditional bacterial culture plating methods. The smaller graph of Fig. 6A is a combined boxplot of these results.

The average output sample concentration was 1.43 x $10^8$ ($\pm 0.15 \log_{10}$) CFU/mL. The goal of the trial runs was that at least 95% of the output sample concentrations would fall within $\pm 0.5 \log_{10}$ of the desired or target concentration 308 of 1.5 x $10^8$ CFU/mL.

Fig. 6B is a table illustrating that 100% of the 41 output sample concentrations were within $\pm 0.5 \log_{10}$ of the desired or target concentration 308 of 1.5 x $10^8$ CFU/mL, 95.1% of the 41 output sample concentrations were within $\pm 0.3 \log_{10}$ of 1.5 x $10^8$ CFU/mL, and 78.0% of the 41 output sample concentrations were within $\pm 0.2 \log_{10}$ of 1.5 x $10^8$ CFU/mL. Since most downstream testing protocols would consider a bacterial concentration error margin of $\pm 0.5 \log_{10}$ to be well within acceptable bounds, the output samples generated from these 41 trial runs can all be used for further downstream testing.

These results show that the method 300 and system 301 (the sensor apparatus 100 and reader 200) disclosed herein is effective at preparing an output sample within acceptable error margins of a desired or target concentration 308. Moreover, these results show that the method 300 and system 301 disclosed herein can also be effective at preparing an output sample within acceptable error margins of a desired or target concentration 308 from a source sample comprising bacteria of a species that was not included in reference bacterial samples 408 used to make the species-agnostic LUT 312. That is, the species-agnostic LUT 312 relied upon to make the output samples is truly "species-agnostic" and has wide applicability to species beyond those used to make the species-agnostic LUT 312.

As will be discussed in the following sections, a large part of the effectiveness of the method 300 and system 301 disclosed herein can be attributed to the aeration protocols disclosed herein.

Figs. 7A and 7B are graphs illustrating the effect of aeration on the bacterial growth rates of a facultative anaerobe (ECo) and a strict aerobe (ABa), respectively. The aerated samples were aerated at a flow rate of about 8.8 $\mu$L per second per mL of the contained sample 113 while the stagnant samples were not aerated. UV-Vis optical density measurements were made over time to track the growth behavior of such samples.

While Fig. 7A shows that aeration has a slight effect on the growth of ECo (the facultative anaerobe), aeration has a much bigger effect on the growth of strict aerobes like ABa, as shown in Fig. 7B. Therefore, aeration provides the dual benefit of reducing the time it takes to prepare a sample preparation times by speeding up the growth of certain types of bacteria (namely strict or obligate aerobes) and making bacterial growth rates more uniform, irrespective of the type of bacteria in the sample.

As previously discussed, it should also be emphasized that too much aeration of the contained sample 113 can have detrimental effects on the ORP signal monitored by the reader 200. Therefore, the contained sample 113 within the sensor apparatus 100 should be aerated at a flow rate within an optimal range. One such range discovered by the applicants is a flow rate between 7.0 $\mu$L per second per mL of the contained sample 113 and 10.0 $\mu$L per second per mL of the contained sample 113. More specifically, the contained sample 113 can be aerated at a flow rate of about 8.8 ($\pm$0.9) $\mu$L per second per mL of the contained sample 113.

In some embodiments, the contained sample 113 within the sensor apparatus 100 can be aerated in accordance with an aeration cycle. The aeration cycle can include an aeration period followed by a non-aerated period where no gas or ambient air is pumped into the container chamber 102. In certain embodiments, the aeration period can be longer than the non-aerated period. For example, the aeration period can be between about 7 minutes and 10 minutes and the non-aerated period can be between about 3 seconds and 10 seconds.

Fig. 8A is a table that shows, once again, that aeration reduces the variance in the growth rates between different species of bacteria. More specifically, the table in Fig. 8A shows that aeration can reduce the overall coefficient of variation (CV) of bacterial doubling times across various species. All samples shown in Fig. 8A were aerated at an aeration flow rate of about 8.8 $\mu$L per second per mL of each sample.

For the three samples containing facultative anaerobic species of bacteria (ECo, SMa, and PVu), the percentage change in bacterial doubling times is in the range of 15% to 21%. However, for the two samples containing strictly aerobic species of bacteria, Aba and PAe, the percentage change in bacterial doubling times is much higher at 41% and 84%, respectively. In addition, when examining the results for all species of bacteria, when such samples are not aerated (or remain stagnant), the CV in their bacterial doubling times is extremely high at 109%. However, with aeration, the CV in their bacterial doubling times drops to 12% and their growth behaviors (as evidenced through their doubling times) becomes very similar. This is important for the success of the species-agnostic method 300 because it ensures that all results are obtained in a similar time frame, irrespective of whether the bacteria in the source sample is a facultative anaerobe or a strict aerobe.

Fig. 8B illustrates that since the overall CV in bacterial doubling times can be reduced using aeration, an average doubling time ($t_{doubling_average}$) can be calculated from a plurality of bacterial doubling times ($t_{doubling}$). For example, an average doubling time ($t_{doubling_average}$) can be calculated by taking an average of at least three bacterial doubling times ($t_{doubling}$). As a more specific example, the table in Fig. 8B shows that the average doubling time ($t_{doubling_average}$) can be calculated by taking an average of five bacterial doubling times ($t_{doubling}$) of bacteria from five different species.

Each of the bacterial doubling times (e.g., the doubling times for ECo, SMa, PVu, Aba, PAe, etc.) can be calculated using O.D. measurements taken of the various reference bacterial samples 408 (see, e.g., Fig. 4). As previously discussed, the O.D. measurements can be converted to bacterial concentrations (in CFU/mL) using a conversion factor. The resulting change in bacterial concentrations can then be plotted as a function of time and such a plot can be fitted to an exponential model such as the one provided in Equation 1 below:

$$N = A(e^{kt}) \qquad \text{[Equation 1]}$$

In Equation 1 above, *N* is the converted bacterial concentration, *t* is the time in minutes, and *A* and *k* are the fit parameters. For purpose of determining the bacterial doubling time ($t_{doubling}$), *A* does not matter since *A* corresponds to the initial concentration of bacteria (i.e., at *t*=0) and is not relevant for determining the bacterial doubling time.

The relationship between the bacterial doubling time ($t_{doubling}$) and k is provided in Equation 2 below:

$$t_{doubling} = \frac{\ln 2}{k} \qquad \text{[Equation 2]}$$

As previously discussed, an average doubling time ($t_{doubling_average}$) can then be calculated from taking an average of the plurality of bacterial doubling times ($t_{doubling}$). The average doubling time ($t_{doubling_average}$) is needed to calculate a time-to-target concentration ($t_{target}$) 320 (see, e.g., Step 300I of Fig. 3) in cases where a desired or target concentration 308 is not included in the species-agnostic LUT 312. For example, a user can input 3.0 x $10^8$ CFU/mL as the desired or target concentration 308, which is beyond any of the species-agnostic bacterial concentrations 316 within the species-agnostic LUT 312 relied upon by the reader 200 (for example, the species-agnostic LUT 312 shown in Fig. 5 only goes up to 1.8 x $10^8$ CFU/mL).

As previously discussed, in certain embodiments, the one or more processors of the reader 200 can opt to calculate the time-to-target concentration 320 even if the desired or target concentration 308 is included in the species-agnostic LUT 312. For example, the one or more processors of the reader 200 can opt for this calculation based on certain heuristics or preset rules that dictate when the threshold ORP change amount 318 from the species-agnostic LUT 312 may be considered too high/too large or may be prone to error. In this case, the one or more processors of the reader can make the determination to calculate the time-to-target concentration 320 rather than rely on certain species-agnostic ORP change amounts 314 from the species-agnostic LUT 312. For example, the one or more processors of the reader 200 can decide that certain smaller species-agnostic ORP change amounts 314 determined earlier on in the ORP monitoring are more accurate or less prone to error than larger species-agnostic ORP change amounts 314 obtained later on as part of the ORP monitoring. The one or more processors reader 200 can make this determination based on real-time or near-real-time analysis of the behavior of the ORP growth curve 311 (for example, if the ORP signal monitored is beginning to flatten out). In this case, the one or more processors of the reader 200 can determine that the smaller species-agnostic ORP change amounts 314 from the species-agnostic LUT 312 are more useful or less prone to error and opt to use such ORP change amounts in calculating the time-to-target concentration 320.

The one or more processors of the reader 200 can be programmed to calculate a time-to-target concentration ($t_{target}$) 320 using Equation 3 below:

$$t_{target} = t_1 + \left(t_{doubling_average} \times \log_2\left(\frac{N_{target}}{N_1}\right)\right) \quad \text{[Equation 3]}$$

In Equation 3 above, $t_{target}$ (or the time-to-target concentration 320) represents the amount of time required for the contained sample 113 to reach the desired or target concentration 308 ($N_{target}$), $N_1$ is a species-agnostic bacterial concentration included in the species-agnostic LUT, $t_1$ represents a time required for the ORP of the contained sample 113 to change by a species-agnostic ORP change amount ($\Delta_{ORP}$) associated with $N_1$ from the species-agnostic LUT 312, and $t_{doubling_average}$ is the average bacterial doubling time. $t_1$ can be determined from real-time ORP monitoring conducted by the reader 200 on the contained sample 113.

For example, Fig. 9A is an ORP growth curve illustrating the change in the ORP of a contained sample 113 measured by the reader 200 over a period of about 60 minutes. For this particular contained sample 113, the user inputted a desired or target concentration 308 of 3.0 x $10^8$ CFU/mL, which is beyond any of the species-agnostic bacterial concentrations 316 within the species-agnostic LUT 312 (for example, the species-agnostic LUT 312 shown in Fig. 5) relied upon by the reader 200. Once the reader 200 determines that the desired or target concentration 308 is not included as part of the species-agnostic LUT 312, the one or more processors of the reader 200 can opt to calculate a time-to-target concentration ($t_{target}$) 320. The one or more processors of the reader 200 can be programmed to calculate the time-to-target concentration ($t_{target}$) 320 using a single paired-entry (i.e., a single species-agnostic bacterial concentration 316 and its associated species-agnostic ORP change amount 314) from the species-agnostic LUT 312 and Equation 3 above.

For example, 1 x $10^8$ CFU/mL (or 1.0E+8) can be selected as $N_1$ from the species-agnostic LUT 312 shown in Fig. 5. The one or more processors of the reader 200 can then monitor the ORP of the contained sample 113 in real-time (see Fig. 9A) to determine $t_1$ based on the amount of time required for the ORP of the contained sample 113 to change by -30 mV (which is the species-agnostic ORP change amount ($\Delta_{ORP}$) associated with $N_1$, see Fig. 5). As shown in Fig. 9A, $t_1$ can be determined as 32 minutes based on the real-time ORP monitoring. Plugging in these values into Equation 3 along with the average doubling time ($t_{doubling_average}$) of 28.4 minutes (see Fig. 8B), the time-to-target concentration ($t_{target}$) 320 can be calculated as 76.4 minutes.

In this example, the reader 200 can alert the user (e.g., a laboratory technician or clinician) that an output sample of the desired or target concentration 308 or an output sample within acceptable error margins of the desired or target concentration 308 has been prepared.

Fig. 9B is a bacterial growth curve illustrating a change in the bacterial concentration within the aforementioned contained sample 113 as a function of time. The bacterial concentration amounts can be obtained by converting O.D. measurements of the aforementioned contained sample 113 over time.

As shown in Fig. 9B, at the 76.4 minute mark, the bacterial concentration within the aforementioned contained sample 113 is about 2.4 x $10^8$ CFU/mL. Since the bacterial concentration of 2.4 x $10^8$ CFU/mL (or 2.4E+8) is within 0.1$\log_{10}$ of the desired or target concentration of 3 x $10^8$ CFU/mL (or 3.0E+8), such a final bacterial concentration is considered well within acceptable error margins (e.g., $\pm$0.5$\log_{10}$) of the desired or target concentration 308.

This example shows the usefulness of the method 300 and system 301 when a desired or target concentration 308 is beyond any bacterial concentrations included as part of the species-agnostic LUT 312. However, as previously mentioned, a time-to-target concentration ($t_{target}$) 320 can also be calculated even if $N_{target}$ is included as part of the species-agnostic LUT 312. For example, a time-to-target concentration ($t_{target}$) 320 can be calculated as long as $N_{target}$ is greater than $N_1$ ($N_{target} > N_1$).

In fact, when an $N_1$ is selected that equals $N_{target}$, the time-to-target concentration ($t_{target}$) 320 simply equals $t_1$. That is, the time-to-target concentration ($t_{target}$) 320 is simply the time required for the ORP of the contained sample 113 to change by the species-agnostic ORP change amount ($\Delta_{ORP}$) associated with $N_1$ from the species-agnostic LUT 312.

It is important to point out that the time-to-target concentration ($t_{target}$) calculations discussed above is only effective since the growth rates of bacteria within all samples (including the contained sample 113 and all reference bacterial samples 408 used to make the species-agnostic LUT 312) have been made more uniform via the specific aeration protocols disclosed herein. That is, the time-to-target concentration ($t_{target}$) calculations discussed above significantly leverages the benefits of aeration in arriving at an accurate end result.

Elements of systems, devices, apparatus, and methods shown with any embodiment are exemplary for the specific embodiment and can be used in combination or otherwise on other embodiments within this disclosure. For example, the steps of any methods depicted in the figures or described in this disclosure do not require the particular order or sequential order shown or described to achieve the desired results. In addition, other steps operations may be provided, or steps or operations may be eliminated or omitted from the described methods or processes to achieve the desired results. Moreover, any components or parts of any apparatus or systems described in this disclosure or depicted in the figures may be removed, eliminated, or omitted to achieve the desired results. In addition, certain components or parts of the systems, devices, or apparatus shown or described herein have been omitted for the sake of succinctness and clarity.

Each of the individual variations or embodiments described and illustrated herein has discrete components and features which may be readily separated from or combined with the features of any of the other variations or embodiments.

Methods recited herein may be carried out in any order of the recited events that is logically possible, as well as the recited order of events. Moreover, additional steps or operations may be provided or steps or operations may be eliminated to achieve the desired result.

Furthermore, where a range of values is provided, every intervening value between the upper and lower limit of that range and any other stated or intervening value in that stated range is encompassed within the invention.

Reference to a singular item, includes the possibility that there are plural of the same items present. More specifically, as used herein and in the appended claims, the singular forms "a," "an," "said" and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Unless defined otherwise, all technical and scientific terms used herein have the same meaning as commonly understood by one of ordinary skill in the art to which this invention belongs.

Reference to the phrase "at least one of", when such phrase modifies a plurality of items or components (or an enumerated list of items or components) means any combination of one or more of those items or components. For example, the phrase "at least one of A, B, and C" means: (i) A; (ii) B; (iii) C; (iv) A, B, and C; (v) A and B; (vi) B and C; or (vii) A and C.

## Claims

**1.** A method (300) of preparing a sample of bacteria of a desired or target concentration or within acceptable error margins of the desired or target concentration, comprising:

Introducing (300B) an aliquot of a sample comprising the bacteria into a sample container, wherein the aliquot of the sample within the sample container is a contained sample in fluid communication with a reference sensor and an active sensor;
incubating and aerating (300D) the contained sample, wherein the contained sample is aerated at a flow rate of between 7.0 microliter, μL, per second per milliliter, mL, of the contained sample and 10.0 μL per second per mL of the contained sample;
monitoring (300E) a change in an oxidation reduction potential, ORP, of the contained sample using a reader electrically coupled to the reference sensor and the active sensor; and
cooling (300K) the contained sample when a concentration of the bacteria in the contained sample is determined (300J) to have reached the desired or target concentration or within acceptable error margins thereof.

**2.** The method (300) of claim 1, further comprising retrieving (300F) a species-agnostic look-up table, LUT, from a database, wherein the species-agnostic LUT comprises species-agnostic ORP change amounts associated with species-agnostic bacterial concentrations, wherein the species-agnostic LUT is generated from a plurality of constituent LUTs comprising ORP change amounts and bacterial concentrations measured using a plurality of reference bacterial samples incubated and aerated at a flow rate of between 7.0 μL per second per mL of each of the reference bacterial samples and 10.0 μL per second per mL of each of the reference bacterial samples.

**3.** The method (300) of claim 2, further comprising:

Selecting (300H) one of the species-agnostic ORP change amounts as a threshold ORP change amount when the species-agnostic ORP change amount selected is associated with one of the species-agnostic bacterial concentrations equal to the desired or target concentration; and
determining (300J) that the concentration of the bacteria in the contained sample has reached the desired or target concentration or within acceptable error margins thereof when the change in the ORP of the contained sample monitored by the reader reaches the threshold ORP change amount.

**4.** The method (300) of claim 2, wherein the species-agnostic LUT is generated from at least three constituent LUTs including a first LUT, a second LUT, and a third LUT; wherein each of the first LUT, the second LUT, or the third LUT is either a species-specific LUT or a strain-specific LUT; wherein the first LUT, the second LUT, and the third LUT are generated using ORP measurements and bacterial concentration measurements made of a first reference bacterial sample, a second reference bacterial sample, and a third reference bacterial sample, respectively; wherein the first reference bacterial sample comprises a bacteria of a first species; wherein the second reference bacterial sample comprises a bacteria of a second species different from the first species; and wherein the third reference bacterial sample comprises a bacteria of a third species different from the second species and the first species.

**5.** The method (300) of claim 4, wherein each of the strain-specific LUTs is generated by:

monitoring a change in the ORP of at least one reference bacterial sample over a period of time;
periodically conducting optical density, OD, measurements of the at least one reference bacterial sample over the same period of time;
converting results of the OD measurements to reference sample bacterial concentrations using a conversion factor; and
associating the reference sample bacterial concentrations with the change in the ORP of the at least one reference bacterial sample.

**6.** The method (300) of claim 2, further comprising:

calculating (3001) a time-to-target concentration, $t_{target}$, representing an amount of time required for the contained sample to reach the desired or target concentration, $N_{target}$, of bacteria using the following relationship:

$$t_{target} = t_1 + \left(t_{doubling_average} \times \log_2\left(\frac{N_{target}}{N_1}\right)\right)$$

wherein $N_{target}$ is not included in the species-agnostic LUT and $N_1$ is a species-agnostic bacterial concentration included in the species-agnostic LUT, wherein $t_1$ represents a time required for the ORP of the contained sample to change by a species-agnostic ORP change amount, $\Delta_{ORP}$, associated with $N_1$ from the species-agnostic LUT, wherein $t_1$ is determined from real-time ORP monitoring conducted by the reader on the contained sample, and wherein $t_{doubling_average}$ is an average bacterial doubling time; and
determining (300J) that the concentration of the bacteria in the contained sample has reached the desired or target concentration or within acceptable error margins thereof when a time elapsed equals the time-to-target concentration.

**7.** The method (300) of claim 2, further comprising:

calculating (3001) a time-to-target concentration, $t_{target}$, representing an amount of time required for the contained sample to reach the desired or target concentration, $N_{target}$, of bacteria using the following relationship:

$$t_{target} = t_1 + \left(t_{doubling_average} \times \log_2\left(\frac{N_{target}}{N_1}\right)\right)$$

wherein $N_{target}$ and $N_1$ are both included in the species-agnostic LUT, wherein $N_{target}$ is greater than $N_1$, $N_{target} > N_1$, wherein $t_1$ represents a time required for the ORP of the contained sample to change by a species-agnostic ORP change amount, ΔORP, associated with $N_1$ from the species-agnostic LUT, wherein $t_1$ is determined from real-time ORP monitoring conducted by the reader on the contained sample, and wherein $t_{doubling-average}$ is an average bacterial doubling time; and
determining (300J) that the concentration of the bacteria in the contained sample has reached the desired or target concentration or within acceptable error margins thereof when a time elapsed equals the time-to-target concentration.

**8.** The method (300) of claim 1, wherein the reference sensor comprises a reference electrode material and a wick in fluid communication with the contained sample such that least some of the contained sample within a chamber cavity of the sample container is drawn by the wick in a direction of the reference electrode material and the contained sample is in fluid contact with the reference electrode material, wherein the active sensor is coupled to at least part of a chamber lateral wall of the sample container, wherein an active electrode material of the active sensor faces the chamber cavity such that the contained sample is in fluid contact with the active electrode material when the contained sample fills the chamber cavity, and wherein the ORP of the contained sample is determined by the reader based on a potential difference measured between the active electrode material and the reference electrode material when the reference sensor and the active sensor are electrically coupled to the reader.

**9.** The method (300) of claim 1, wherein the bacteria is a facultative anaerobe or a strict aerobe.

**10.** The method (300) of claim 1, wherein the sample of bacteria of the desired or target concentration is prepared without any prior knowledge of a species of the bacteria in the contained sample or previously ascertaining a species of the bacteria in the contained sample.

**11.** The method (300) of claim 1, wherein the desired or target concentration is between 1.4 x $10^8$ CFU/mL and 1.6 x $10^8$ CFU/mL.

**12.** The method (300) of claim 1, wherein the acceptable error margins are $\pm 0.510g_{10}$.

**13.** The method (300) of claim 1, further comprising diluting (300A) a source sample comprising the bacteria by a dilution factor between 1:10 and 1:100 to yield a diluted sample; and wherein the aliquot of the sample introduced into the sample container is an aliquot of the diluted sample.

**14.** The method (300) of claim 1, wherein the contained sample is aerated (300D) in accordance with an aeration cycle, wherein the aeration cycle comprises an aeration period followed by a non-aerated period, wherein the aeration period is longer than the non-aerated period, and wherein the aeration period is between about 7 minutes and 10 minutes and the non-aerated period is between about 3 seconds and 10 seconds.

**15.** The method (300) of claim 1, wherein aerating (300D) the contained sample further comprises pumping ambient air into the sample container through an opening defined along a base of the sample container using a motorized piston pump housed within the reader.

## Patentansprüche

**1.** Verfahren (300) zum Herstellen einer Probe von Bakterien mit einer gewünschten oder Zielkonzentration oder innerhalb akzeptabler Fehlergrenzen der gewünschten oder Zielkonzentration, umfassend:

Einbringen (300B) eines Aliquot einer die Bakterien umfassenden Probe in einen Probenbehälter, wobei das Aliquot der Probe in dem Probenbehälter eine enthaltene Probe ist, die in Fluidverbindung mit einem Referenzsensor und einem aktiven Sensor steht;
Inkubieren und Belüften (300D) der enthaltenen Probe, wobei die enthaltene Probe mit einer Durchflussrate zwischen 7,0 Mikrolitern, µL, pro Sekunde pro Milliliter, mL, der enthaltenen Probe und 10,0 µL pro Sekunde pro mL der enthaltenen Probe belüftet wird;
Überwachen (300E) einer Änderung des Oxidations-Reduktions-Potenzials, ORP, der enthaltenen Probe unter Verwendung eines Lesegeräts, das elektrisch mit dem Referenzsensor und dem aktiven Sensor gekoppelt ist; und
Kühlen (300K) der enthaltenen Probe, wenn bestimmt wird (300J), dass eine Konzentration der Bakterien in der enthaltenen Probe die gewünschte oder Zielkonzentration oder eine innerhalb akzeptabler Fehlergrenzen davon liegende Konzentration erreicht hat.

**2.** Verfahren (300) nach Anspruch 1, das ferner ein Abrufen (300F) einer artenunabhängigen Nachschlagetabelle, LUT, aus einer Datenbank umfasst, wobei die artenunabhängige LUT artenunabhängige ORP-Änderungsbeträge umfasst, die mit artenunabhängigen Bakterienkonzentrationen verbunden sind, wobei die artenunabhängige LUT aus einer Mehrzahl von konstituierenden LUTs erzeugt wird, die ORP-Änderungsbeträge und Bakterienkonzentrationen umfassen, die unter Verwendung einer Mehrzahl von Referenzbakterienproben gemessen wurden, die bei einer Durchflussrate zwischen 7,0 µL pro Sekunde pro mL jeder der Referenzbakterienproben und 10,0 µL pro Sekunde pro mL jeder der Referenzbakterienproben inkubiert und belüftet wurden.

**3.** Verfahren (300) nach Anspruch 2, ferner umfassend:

Auswählen (300H) einer der artenunabhängigen ORP-Änderungsbeträge als Schwellenwert-ORP-Änderungsbetrag, wenn der ausgewählte artenunabhängige ORP-Änderungsbetrag mit einer der artenunabhängigen Bakterienkonzentrationen assoziiert ist, die der gewünschten oder Zielkonzentration entspricht; und

Bestimmen (300J), dass die Konzentration der Bakterien in der enthaltenen Probe die gewünschte oder Zielkonzentration oder die innerhalb akzeptabler Fehlergrenzen davon liegende Konzentration erreicht hat, wenn die vom Lesegerät überwachte Änderung des ORP der enthaltenen Probe den Schwellenwert für die ORP-Änderung erreicht.

4. Verfahren (300) nach Anspruch 2, wobei die artenunabhängige LUT aus mindestens drei konstituierenden LUTs erzeugt wird, darunter eine erste LUT, eine zweite LUT und eine dritte LUT; wobei jede der ersten LUT, der zweiten LUT oder der dritten LUT entweder eine artspezifische LUT oder eine stammspezifische LUT ist; wobei die erste LUT, die zweite LUT und die dritte LUT unter Verwendung von ORP-Messungen und Bakterienkonzentrationsmessungen erzeugt werden, die an einer ersten Referenzbakterienprobe, einer zweiten Referenzbakterienprobe bzw. einer dritten Referenzbakterienprobe durchgeführt werden; wobei die erste Referenzbakterienprobe ein Bakterium einer ersten Spezies umfasst; wobei die zweite Referenzbakterienprobe ein Bakterium einer zweiten Spezies umfasst, die sich von der ersten Spezies unterscheidet; und wobei die dritte Referenzbakterienprobe ein Bakterium einer dritten Spezies umfasst, die sich von der zweiten Spezies und der ersten Spezies unterscheidet.

5. Verfahren (300) nach Anspruch 4, wobei jede der stammspezifischen LUTs erzeugt wird durch:

Überwachen einer Änderung des ORP mindestens einer Referenzbakterienprobe über einen Zeitraum;

periodisches Durchführen von Messungen der optischen Dichte, OD, der mindestens einen Referenzbakterienprobe über denselben Zeitraum;

Umwandeln von Ergebnissen der OD-Messungen in Referenzproben-Bakterienkonzentrationen unter Verwendung eines Umwandlungsfaktors; und

Zuordnen der Referenzproben-Bakterienkonzentrationen zur Änderung des ORP der mindestens einen Referenzbakterienprobe.

6. Verfahren (300) nach Anspruch 2, ferner umfassend:

Berechnen (300I) einer Zeit bis zur Zielkonzentration, $t_{Ziel}$, die eine Zeitspanne darstellt, die erforderlich ist, damit die enthaltene Probe die gewünschte oder Zielkonzentration, $N_{Ziel}$, an Bakterien erreicht, unter Verwendung der folgenden Beziehung:

$$t_{Ziel} = t_1 + \left( t_{doppel_Durchschnitt} \times \log_2 \left( \frac{N_{Ziel}}{N_1} \right) \right)$$

wobei $N_{Ziel}$ nicht in der artenunabhängigen LUT enthalten ist und $N_1$ eine artenunabhängige Bakterienkonzentration ist, die in der artenunabhängigen LUT enthalten ist, wobei $t_1$ die Zeit darstellt, die erforderlich ist, damit sich das ORP der enthaltenen Probe um einen artenunabhängigen ORP-Änderungsbetrag, $\Delta_{ORP}$, ändert, der mit $N_1$ aus der artenunabhängigen LUT verbunden ist, wobei $t_1$ aus der Echtzeit-ORP-Überwachung bestimmt wird, die vom Lesegerät an der enthaltenen Probe durchgeführt wird, und wobei $t_{doppel_Durchschnitt}$ eine durchschnittliche Bakterienverdopplungszeit ist; und

Bestimmen (300J), dass die Konzentration der Bakterien in der enthaltenen Probe die gewünschte oder Zielkonzentration oder die innerhalb akzeptabler Fehlergrenzen davon liegende Konzentration erreicht hat, wenn eine verstrichene Zeit der Zeit bis zur Zielkonzentration entspricht.

7. Verfahren (300) nach Anspruch 2, ferner umfassend:

Berechnen (300I) einer Zeit bis zur Zielkonzentration, $t_{Ziel}$, die eine Zeitdauer darstellt, die erforderlich ist, damit die enthaltene Probe die gewünschte oder Zielkonzentration, $N_{Ziel}$, von Bakterien erreicht, unter Verwendung der folgenden Beziehung:

$$t_{Ziel} = t_1 + \left( t_{doppel_Durchschnitt} \times \log_2 \left( \frac{N_{Ziel}}{N_1} \right) \right)$$

wobei $N_{Ziel}$ und $N_1$ beide in der artenunabhängigen LUT enthalten sind, wobei $N_{Ziel}$ größer ist als $N_1$, $N_{Ziel} > N_1$, wobei $t_1$ eine Zeit darstellt, die erforderlich ist, damit sich das ORP der enthaltenen Probe um einen artenunabhängigen ORP-Änderungsbetrag, ΔORP, ändert, der mit $N_1$ aus der artenunabhängigen LUT verbunden ist, wobei $t_1$ aus der Echtzeit-ORP-Überwachung bestimmt wird, die vom Lesegerät an der enthaltenen Probe durchgeführt wird, und wobei $t_{doppel_Durchschnitt}$ eine durchschnittliche Bakterienverdopplungszeit ist; und Bestimmen (300J), dass die Konzentration der Bakterien in der enthaltenen Probe die gewünschte oder Zielkonzentration oder eine innerhalb akzeptabler Fehlergrenzen davon liegende Konzentration erreicht hat, wenn eine verstrichene Zeit der Zeit bis zur Zielkonzentration entspricht.

**8.** Verfahren (300) nach Anspruch 1, wobei der Referenzsensor ein Referenzelektrodenmaterial und einen Docht umfasst, der in Fluidverbindung mit der enthaltenen Probe steht, so dass zumindest ein Teil der enthaltenen Probe innerhalb eines Kammerhohlraums des Probenbehälters durch den Docht in Richtung des Referenzelektrodenmaterials gezogen wird und die enthaltene Probe in Fluidkontakt mit dem Referenzelektrodenmaterial steht, wobei der aktive Sensor mit mindestens einem Teil einer Kammerseitenwand des Probenbehälters gekoppelt ist, wobei ein aktives Elektrodenmaterial des aktiven Sensors der Kammerhöhle zugewandt ist, so dass die enthaltene Probe in Fluidkontakt mit dem aktiven Elektrodenmaterial steht, wenn die enthaltene Probe die Kammerhöhle füllt, und wobei das ORP der enthaltenen Probe durch das Lesegerät auf der Grundlage einer zwischen dem aktiven Elektrodenmaterial und dem Referenzelektrodenmaterial gemessenen Potentialdifferenz bestimmt wird, wenn der Referenzsensor und der aktive Sensor elektrisch mit dem Lesegerät gekoppelt sind.

**9.** Verfahren (300) nach Anspruch 1, wobei die Bakterien fakultativ anaerob oder streng aerob sind.

**10.** Verfahren (300) nach Anspruch 1, wobei die Probe von Bakterien der gewünschten oder Zielkonzentration ohne vorherige Kenntnis einer Spezies der Bakterien in der enthaltenen Probe oder ohne vorherige Feststellung einer Spezies der Bakterien in der enthaltenen Probe hergestellt wird.

**11.** Verfahren (300) nach Anspruch 1, wobei die gewünschte oder Zielkonzentration zwischen $1{,}4 \times 10^8$ CFU/mL und $1{,}6 \times 10^8$ CFU/mL liegt.

**12.** Verfahren (300) nach Anspruch 1, wobei die akzeptablen Fehlergrenzen $\pm 0{,}5 \log_{10}$ betragen.

**13.** Verfahren (300) nach Anspruch 1, das ferner ein Verdünnen (300A) einer die Bakterien umfassenden Ausgangsprobe mit einem Verdünnungsfaktor zwischen 1:10 und 1:100 umfasst, um eine verdünnte Probe zu erhalten; und wobei das in den Probenbehälter eingeführte Aliquot der Probe ein Aliquot der verdünnten Probe ist.

**14.** Verfahren (300) nach Anspruch 1, wobei die enthaltene Probe gemäß einem Belüftungszyklus belüftet wird (300D), wobei der Belüftungszyklus eine Belüftungsperiode gefolgt von einer nicht belüfteten Periode umfasst, wobei die Belüftungsperiode länger ist als die nicht belüftete Periode, und wobei die Belüftungsperiode zwischen etwa 7 Minuten und 10 Minuten und die nicht belüftete Periode zwischen etwa 3 Sekunden und 10 Sekunden beträgt.

**15.** Verfahren (300) nach Anspruch 1, wobei das Belüften (300D) der enthaltenen Probe ferner ein Pumpen von Umgebungsluft in den Probenbehälter durch eine entlang einer Basis des Probenbehälters definierte Öffnung unter Verwendung einer in dem Lesegerät untergebrachten motorisierten Kolbenpumpe umfasst.

## Revendications

**1.** Procédé (300) de préparation d'un échantillon de bactérie d'une concentration souhaitée ou cible ou dans des marges d'erreur acceptables par rapport à la concentration souhaitée ou cible, comprenant:

l'introduction (300B) d'une aliquote d'un échantillon comprenant les bactéries dans un récipient à échantillon, dans lequel l'aliquote de l'échantillon dans le récipient à échantillon est un échantillon contenu en communication fluidique avec un capteur de référence et un capteur actif;
l'incubation et l'aération (300D) de l'échantillon contenu, dans lequel l'échantillon contenu est aéré à un débit compris entre 7,0 microlitres, µL, par seconde par millilitre, mL, de l'échantillon contenu et 10,0 µL par seconde par mL de l'échantillon contenu;
la surveillance (300E) d'une variation du potentiel d'oxydoréduction, rH, de l'échantillon contenu à l'aide d'un lecteur couplé électriquement au capteur de référence et au capteur actif; et

le refroidissement (300 K) de l'échantillon contenu lorsqu'il est déterminé (300J) que la concentration des bactéries dans l'échantillon contenu a atteint la concentration souhaitée ou cible ou se situe dans les marges d'erreur acceptables de celle-ci.

**2.** Procédé (300) selon la revendication 1, comprenant en outre la récupération (300F) d'une table de consultation indépendante de l'espèce, LUT, à partir d'une base de données, dans lequel la LUT indépendante de l'espèce comprend des quantités de variation de rH indépendantes de l'espèce associées à des concentrations bactériennes indépendantes de l'espèce, dans lequel la LUT est générée à partir d'une pluralité de LUT constitutives comprenant des quantités de variation de rH et des concentrations bactériennes mesurées à l'aide d'une pluralité d'échantillons de bactérie de référence incubés et aérés à un débit compris entre 7,0 $\mu$L par seconde par mL de chacun des échantillons de bactérie de référence et 10,0 $\mu$L par seconde par mL de chacun des échantillons de bactérie de référence.

**3.** Procédé (300) selon la revendication 2, comprenant en outre:

la sélection (300H) de l'une des quantités de variation de rH indépendantes de l'espèce comme quantité de variation de rH seuil lorsque la quantité de variation de rH indépendante de l'espèce sélectionnée est associée à l'une des concentrations bactériennes indépendantes de l'espèce égales à la concentration souhaitée ou cible; et
la détermination (300J) que la concentration des bactéries dans l'échantillon contenu a atteint la concentration souhaitée ou cible ou se situe dans les marges d'erreur acceptables de celle-ci lorsque la variation de rH de l'échantillon contenu surveillé par le lecteur atteint la quantité de variation seuil rH.

**4.** Procédé (300) selon la revendication 2, dans lequel la LUT indépendante de l'espèce est générée à partir d'au moins trois LUT constitutives comprenant une première LUT, une deuxième LUT et une troisième LUT; dans lequel chacune des première, deuxième et troisième LUT est soit une LUT spécifique à une espèce, soit une LUT spécifique à une souche; dans lequel la première LUT, la deuxième LUT et la troisième LUT sont générées à l'aide de mesures rH et de mesures de concentration bactérienne effectuées respectivement sur un premier échantillon de bactérie de référence, un deuxième échantillon de bactérie de référence et un troisième échantillon de bactérie de référence; dans lequel le premier échantillon de bactérie de référence comprend une bactérie d'une première espèce; dans lequel le deuxième échantillon bactérien de référence comprend une bactérie d'une deuxième espèce différente de la première espèce; et dans lequel le troisième échantillon bactérien de référence comprend une bactérie d'une troisième espèce différente de la deuxième espèce et de la première espèce.

**5.** Procédé (300) selon la revendication 4, dans lequel chacune des LUT spécifiques à une souche est générée par:

surveillance d'une variation de rH d'au moins un échantillon de bactérie de référence sur une période donnée;
réalisation périodique de mesures de densité optique, DO, sur l'au moins un échantillon de bactérie de référence pendant la même période;
conversion des résultats des mesures de DO en concentrations bactériennes d'échantillon de référence à l'aide d'un facteur de conversion; et
association des concentrations bactériennes d'échantillon de référence au rH de l'au moins un échantillon bactérien de référence.

**6.** Procédé (300) selon la revendication 2, comprenant en outre:

le calcul (300I) d'un temps nécessaire pour atteindre la concentration cible, $t_{target}$, représentant le temps nécessaire pour que l'échantillon contenu atteigne la concentration souhaitée ou cible, $N_{target}$, de bactéries à l'aide de la relation suivante:

$$t_{target} = t_1 + \left( t_{doubling_average} \times \log_2 \left( \frac{N_{target}}{N_1} \right) \right)$$

dans lequel $N_{target}$ n'est pas inclus dans la LUT indépendante de l'espèce et $N_1$ est une concentration bactérienne indépendante de l'espèce incluse dans la LUT indépendante de l'espèce, dans lequel $t_1$ représente le temps nécessaire pour que le rH de l'échantillon contenu change d'une quantité de variation de rH indépendante de l'espèce, $\Delta_{rH}$, associé à $N_1$ à partir de la LUT indépendante de l'espèce, dans lequel $t_1$ est déterminé à partir d'une surveillance en temps réel de rH effectuée par le lecteur sur l'échantillon contenu, et dans lequel $t_{doubling_average}$

est un temps de doublement bactérien moyen; et
la détermination (300J) que la concentration des bactéries dans l'échantillon contenu a atteint la concentration souhaitée ou cible ou se situe dans les marges d'erreur acceptables de celle-ci lorsqu'un temps écoulé est égal au temps nécessaire pour atteindre la concentration cible.

**7.** Procédé (300) selon la revendication 2, comprenant en outre:

le calcul (300I) d'un temps nécessaire pour atteindre la concentration cible, $t_{target}$, représentant le temps nécessaire pour que l'échantillon contenu atteigne la concentration souhaitée ou cible, $N_{target}$, de bactéries à l'aide de la relation suivante:

$$t_{target} = t_1 + \left( t_{doubling_average} \times \log_2 \left( \frac{N_{target}}{N_1} \right) \right)$$

dans lequel $N_{target}$ et $N_1$ sont tous les deux inclus dans la LUT indépendante de l'espèce, dans lequel $N_{target}$ est supérieur à $N_1$, $N_{target} > N_1$, dans lequel $t_1$ représente le temps nécessaire pour que le rH de l'échantillon contenu change d'une quantité de variation de rH indépendante de l'espèce, $\Delta_{rH}$, associé à $N_1$ à partir de la LUT indépendante de l'espèce, dans lequel $t_1$ est déterminé à partir d'une surveillance en temps réel de rH effectuée par le lecteur sur l'échantillon contenu, et dans lequel $t_{doubling_average}$ est un temps de doublement bactérien moyen; et
la détermination (300J) que la concentration des bactéries dans l'échantillon contenu a atteint la concentration souhaitée ou cible ou se situe dans les marges d'erreur acceptables de celle-ci lorsqu'un temps écoulé est égal au temps nécessaire pour atteindre la concentration cible.

**8.** Procédé (300) selon la revendication 1, dans lequel le capteur de référence comprend un matériau d'électrode de référence et une mèche en communication fluidique avec l'échantillon contenu, de telle sorte qu'au moins une partie de l'échantillon contenu dans une cavité de chambre du récipient à échantillon soit aspirée par la mèche dans la direction du matériau d'électrode de référence et que l'échantillon contenu soit en contact fluidique avec le matériau d'électrode de référence, dans lequel le capteur actif est couplé à au moins une partie d'une paroi latérale de la chambre du récipient à échantillon, dans lequel un matériau d'électrode active du capteur actif fait face à la cavité de la chambre de telle sorte que l'échantillon contenu soit en contact fluidique avec le matériau d'électrode actif lorsque l'échantillon contenu remplit la cavité de la chambre, et dans lequel le rH de l'échantillon contenu est déterminé par le lecteur sur la base d'une différence de potentiel mesurée entre le matériau d'électrode actif et le matériau d'électrode de référence lorsque le capteur de référence et le capteur actif sont couplés électriquement au lecteur.

**9.** Procédé (300) selon la revendication 1, dans lequel la bactérie est un anaérobie facultatif ou un aérobie strict.

**10.** Procédé (300) selon la revendication 1, dans lequel l'échantillon de bactéries de la concentration souhaitée ou cible est préparé sans aucune connaissance préalable d'une espèce de bactéries dans l'échantillon contenu ni vérification préalable d'une espèce de bactéries dans l'échantillon contenu.

**11.** Procédé (300) selon la revendication 1, dans laquelle la concentration souhaitée ou cible est comprise entre $1,4 \times 10^8$ CFU/mL et $1,6 \times 10^8$ CFU/mL.

**12.** Procédé (300) selon la revendication 1, dans lequel les marges d'erreur acceptables sont de $\pm 0,5 \log_{10}$.

**13.** Procédé (300) selon la revendication 1, comprenant en outre la dilution (300A) d'un échantillon source comprenant les bactéries par un facteur de dilution compris entre 1:10 et 1:100 pour obtenir un échantillon dilué; et dans lequel l'aliquote de l'échantillon introduit dans le récipient d'échantillon est une aliquote de l'échantillon dilué.

**14.** Procédé (300) selon la revendication 1, dans lequel l'échantillon contenu est aéré (300D) conformément à un cycle d'aération, dans lequel le cycle d'aération comprend une période d'aération suivie d'une période de non aération, dans lequel la période d'aération est plus longue que la période de non aération, et dans lequel la période d'aération est comprise entre environ 7 minutes et 10 minutes et la période de non aération est comprise entre environ 3 secondes et 10 secondes.

**15.** Procédé (300) selon la revendication 1, dans lequel l'aération (300D) de l'échantillon contenu comprend en outre le pompage d'air ambiant dans le récipient à échantillon par une ouverture pratiquée le long de la base du récipient à échantillon à l'aide d'une pompe à piston motorisée logée à l'intérieur du lecteur.

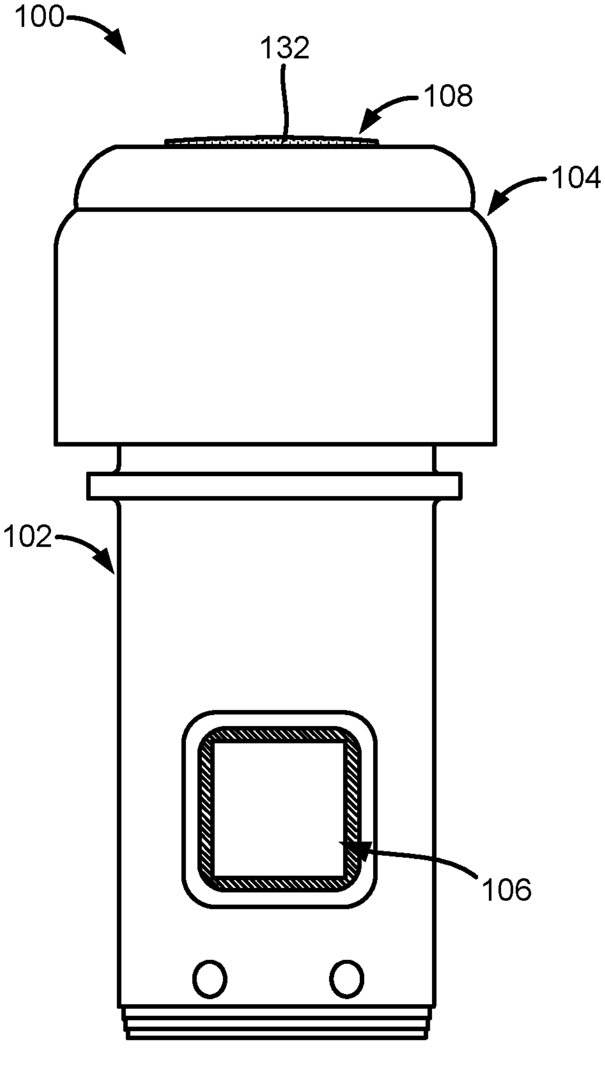


FIG. 1A

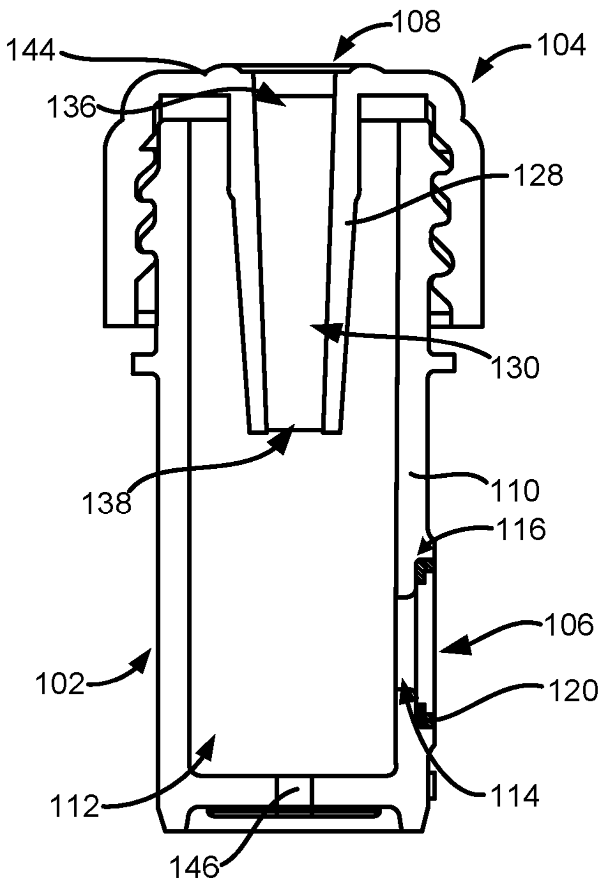


FIG. 1B

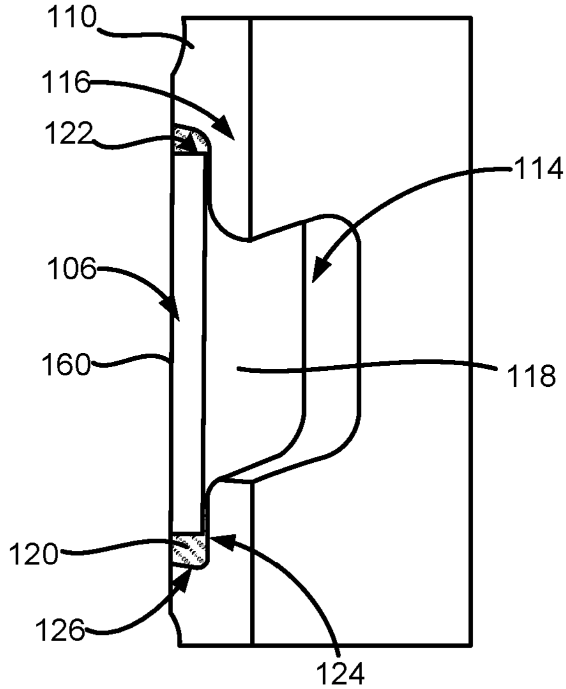


FIG. 1C

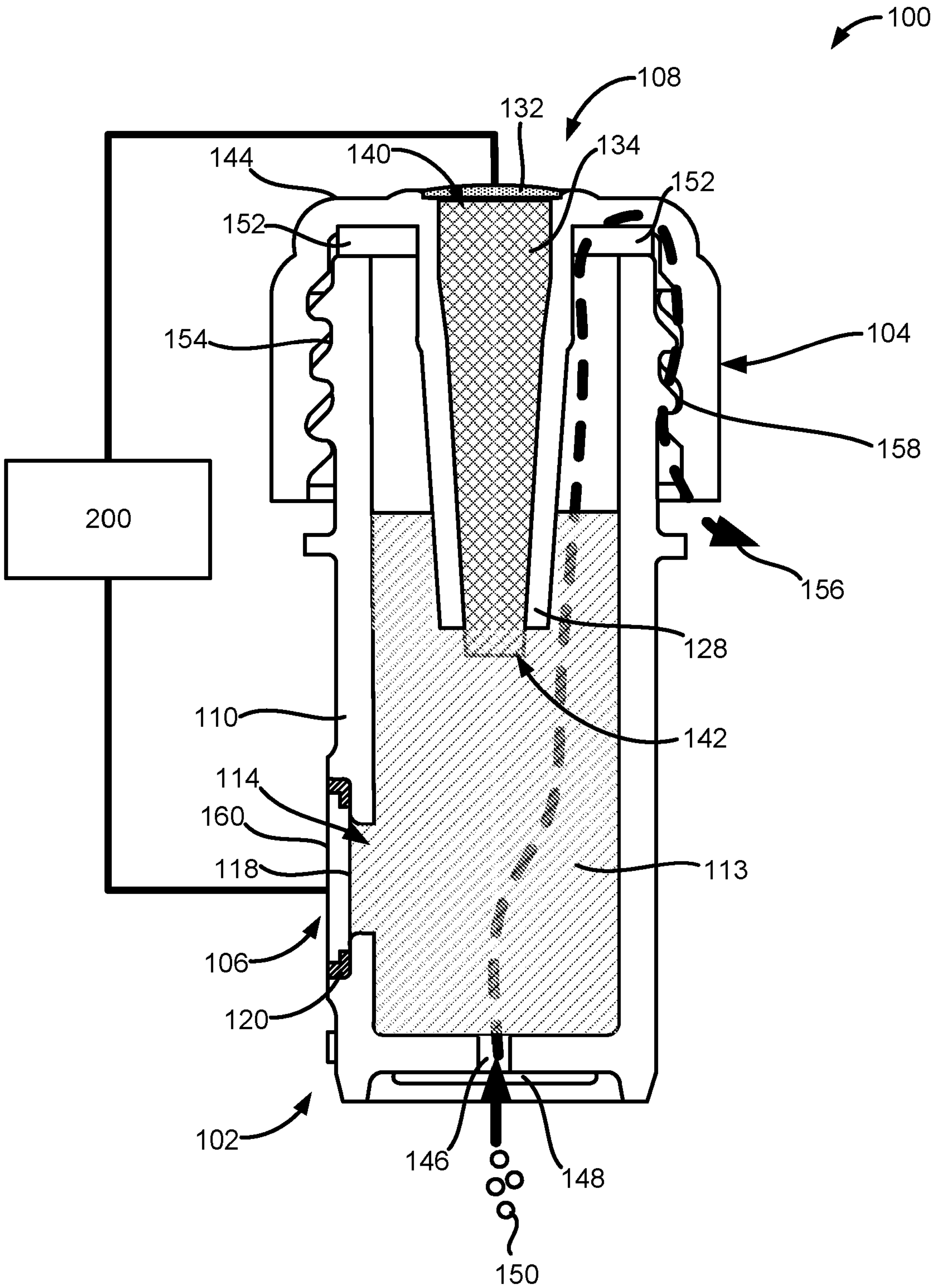
100
108
132
134
144
140
152
152
154
104
158
200
156
128
142
110
114
160
118
113
106
120
102
146
148
150

FIG. 1D

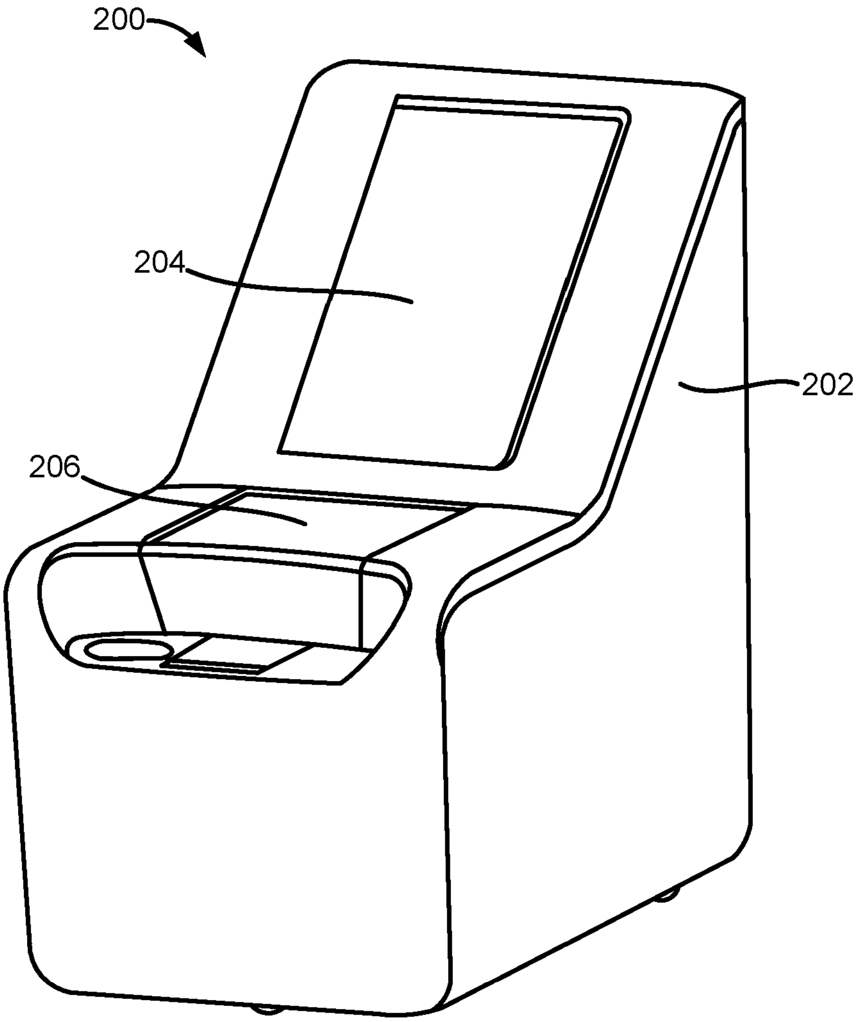


FIG. 2A

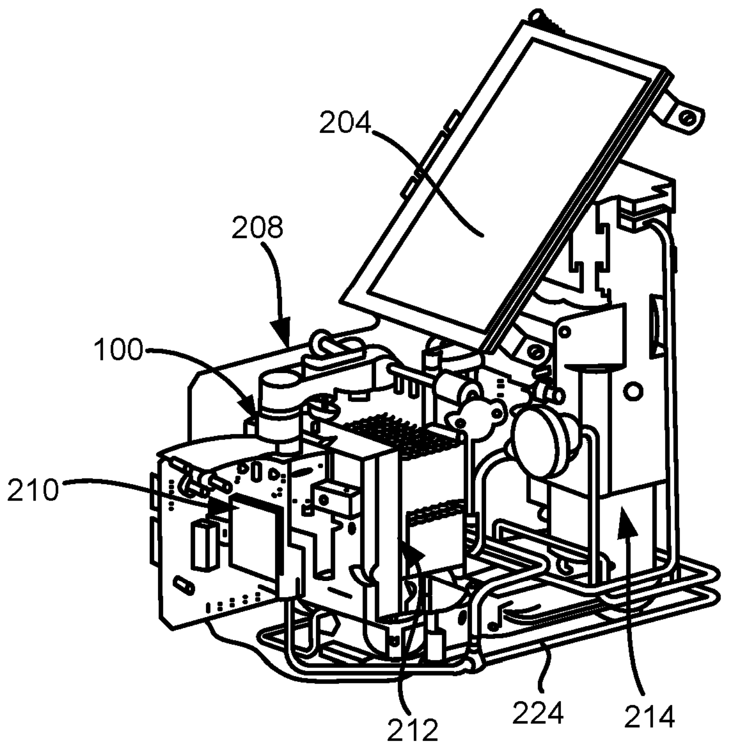


FIG. 2B

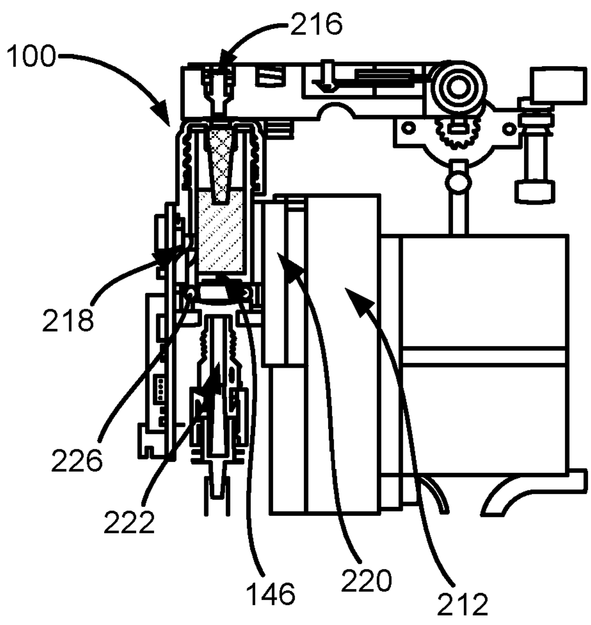


FIG. 2C

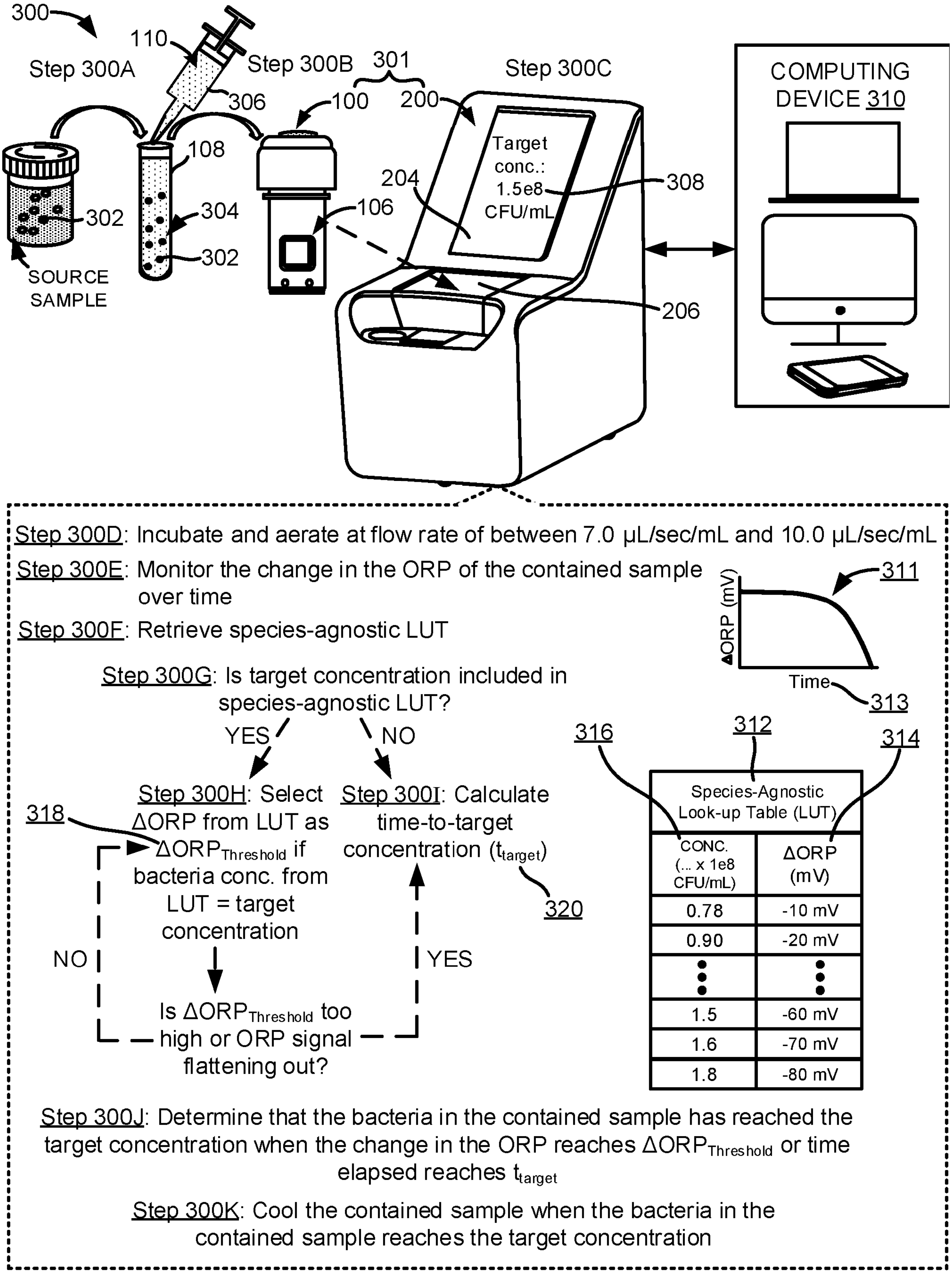
300
110
Step 300A
Step 300B
301
Step 300C
COMPUTING DEVICE 310
306
100
200
108
Target conc.: 1.5e8 CFU/mL
204
308
302
304
106
302
SOURCE SAMPLE
206
Step 300D: Incubate and aerate at flow rate of between 7.0 µL/sec/mL and 10.0 µL/sec/mL
Step 300E: Monitor the change in the ORP of the contained sample over time
311
ΔORP (mV)
Step 300F: Retrieve species-agnostic LUT
Step 300G: Is target concentration included in species-agnostic LUT?
Time
313
YES
NO
316
312
314
Step 300H: Select ΔORP from LUT as ΔORPThreshold if bacteria conc. from LUT = target concentration
Step 300I: Calculate time-to-target concentration (ttarget)
318
320
Species-Agnostic Look-up Table (LUT)
CONC. (... x 1e8 CFU/mL)
ΔORP (mV)
0.78
-10 mV
0.90
-20 mV
1.5
-60 mV
1.6
-70 mV
1.8
-80 mV
NO
YES
Is ΔORPThreshold too high or ORP signal flattening out?
Step 300J: Determine that the bacteria in the contained sample has reached the target concentration when the change in the ORP reaches ΔORPThreshold or time elapsed reaches ttarget
Step 300K: Cool the contained sample when the bacteria in the contained sample reaches the target concentration

FIG. 3

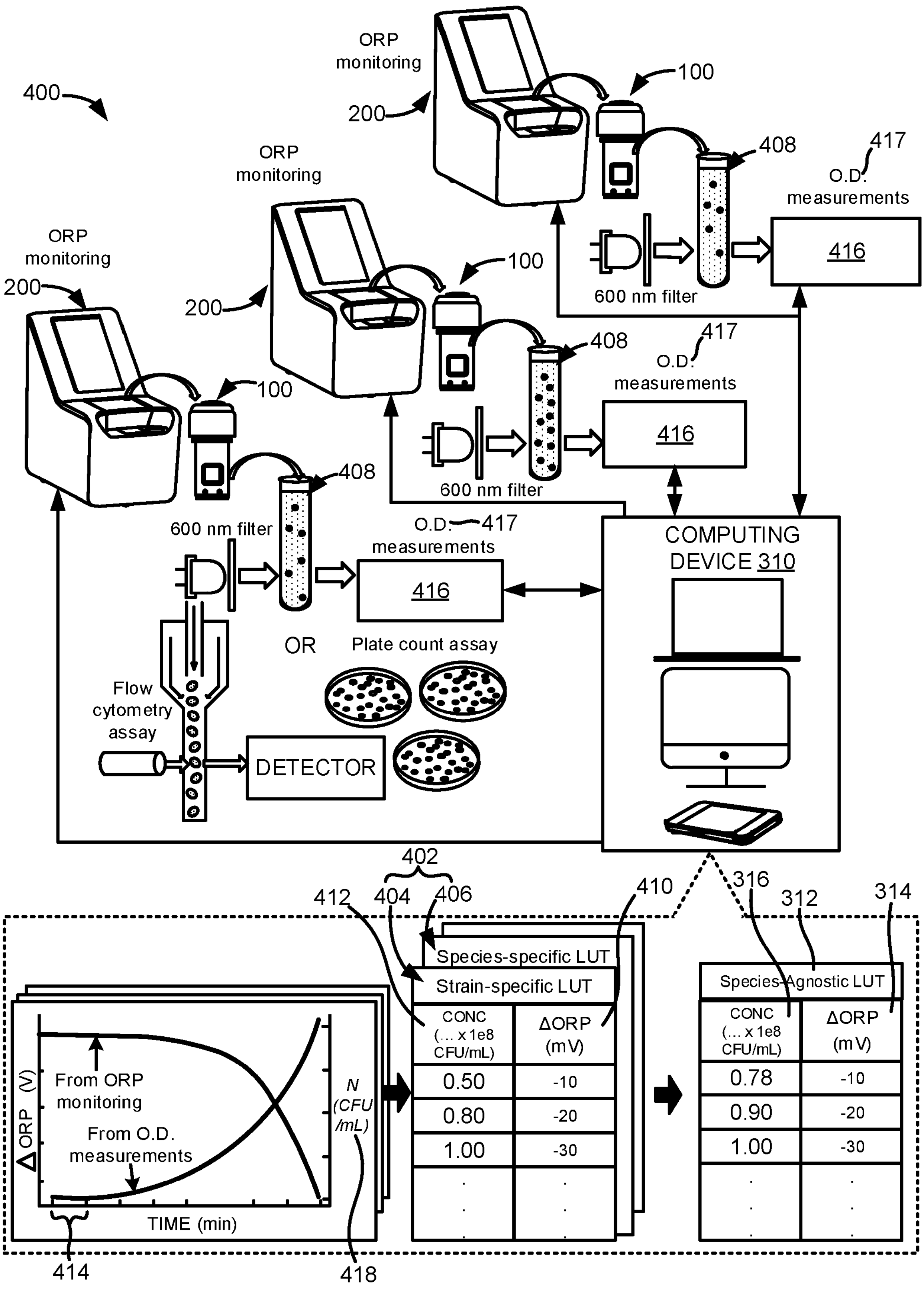
400
ORP monitoring
200
100
408
417
O.D. measurements
416
600 nm filter
COMPUTING DEVICE 310
OR
Plate count assay
Flow cytometry assay
DETECTOR
402
412
404
406
410
316
312
314
Species-specific LUT
Strain-specific LUT
CONC (… x 1e8 CFU/mL)
ΔORP (mV)
0.50
-10
0.80
-20
1.00
-30
Species-Agnostic LUT
0.78
0.90
ΔORP (V)
From ORP monitoring
From O.D. measurements
N (CFU/mL)
TIME (min)
414
418

FIG. 4

404 312

410 412 316 314

| ΔORP (mV) | Bacterial Concentration (*N in* CFU/ml) | | | | | | Species Agnostic Concentrations (Avg. Concentrations + SD in CFU/mL) | ΔORP (mV) |
|---|---|---|---|---|---|---|---|---|
| | **ECo PSC-91** | **KPn PSC-38** | **ABa UCLA-126** | **PAe PSC-30** | **PVu UCLA-32** | **SMa CDC-91** | | |
| -10 | 6.6E+07 | 1.0E+08 | 6.4E+07 | 1.3E+08 | 4.6E+07 | 5.9E+07 | 7.8E+07$\pm$ 0.16og$_{10}$ | -10 |
| -20 | 7.5E+07 | 1.1E+08 | 7.4E+07 | 1.5E+08 | 5.3E+07 | 8.0E+07 | 9.0E+07 $\pm$ 0.16log$_{10}$ | -20 |
| -30 | 8.2E+07 | 1.2E+08 | 8.7E+07 | 1.7E+08 | 6.1E+07 | 1.0E+08 | 1.0E+08 $\pm$ 0.15log$_{10}$ | -30 |
| -40 | 8.9E+07 | 1.3E+08 | 1.1E+08 | 1.8E+08 | 6.8E+07 | 1.1E+08 | 1.2E+08 $\pm$ 0.14log$_{10}$ | -40 |
| -50 | 9.5E+07 | 1.4E+08 | 1.4E+08 | 2.0E+08 | 7.5E+07 | 1.2E+08 | 1.3E+08 $\pm$ 0.15log$_{10}$ | -50 |
| -60 | 1.0E+08 | 1.6E+08 | 1.8E+08 | 2.3E+08 | 8.2E+07 | 1.3E+08 | 1.5E+08 $\pm$ 0.17log$_{10}$ | -60 |
| -70 | 1.1E+08 | 1.7E+08 | 2.1E+08 | 2.5E+08 | 9.4E+07 | 1.4E+08 | 1.6E+08 $\pm$ 0.16log$_{10}$ | -70 |
| -80 | 1.2E+08 | 1.9E+08 | 2.5E+08 | 2.8E+08 | 1.1E+08 | 1.5E+08 | 1.8E+08 $\pm$ 0.17log$_{10}$ | -80 |

**FIG. 5**

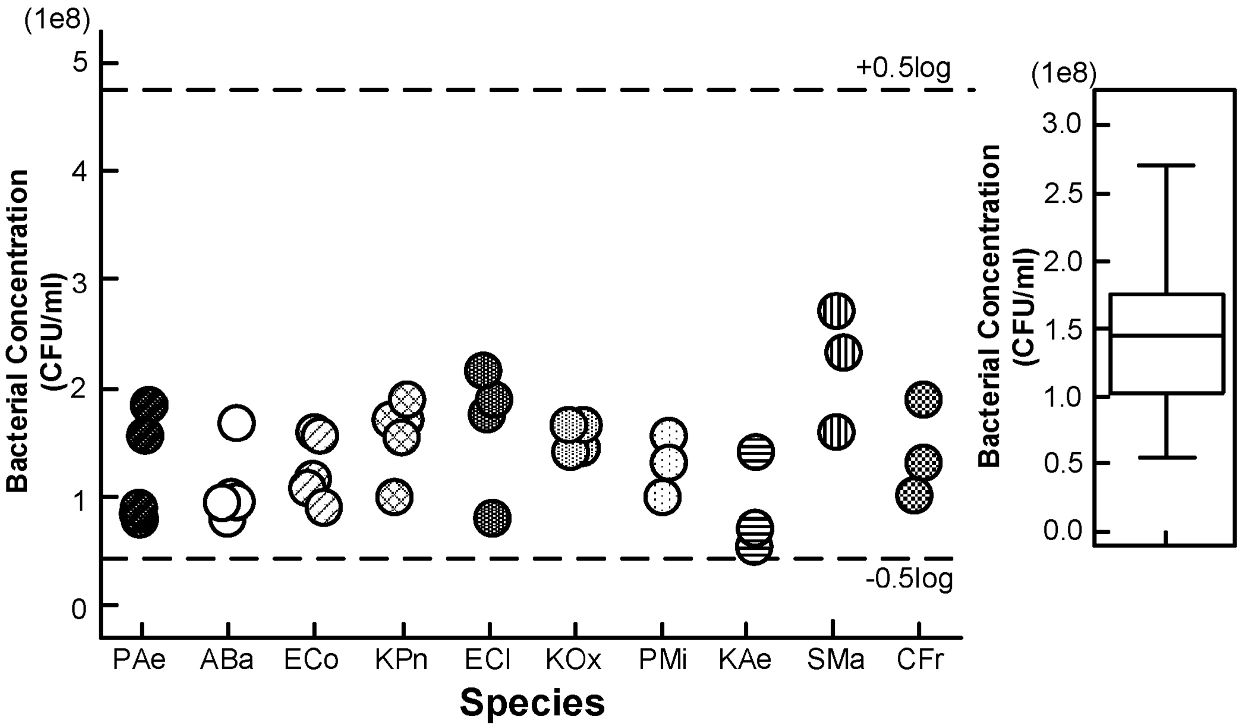


FIG. 6A

| Target Bacterial Concentration Range | Output Samples in Range (%) | Output Samples in Range (# out of 41 total) |
|---|---|---|
| 1.5E+08 ± 0.2$\log_{10}$ | 78.0 | 32/41 |
| 1.5E+08 ± 0.3$\log_{10}$ | 95.1 | 39/41 |
| 1.5E+08 ± 0.5$\log_{10}$ | 100 | 41/41 |

FIG. 6B

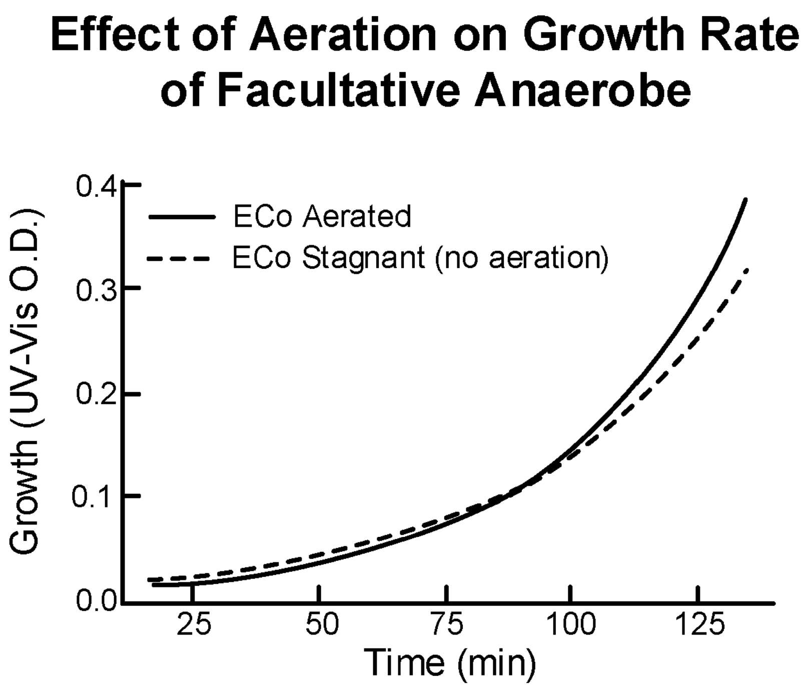
Effect of Aeration on Growth Rate
of Facultative Anaerobe
ECo Aerated
ECo Stagnant (no aeration)
Growth (UV-Vis O.D.)
0.0
0.1
0.2
0.3
0.4
25
50
75
100
125
Time (min)

FIG. 7A

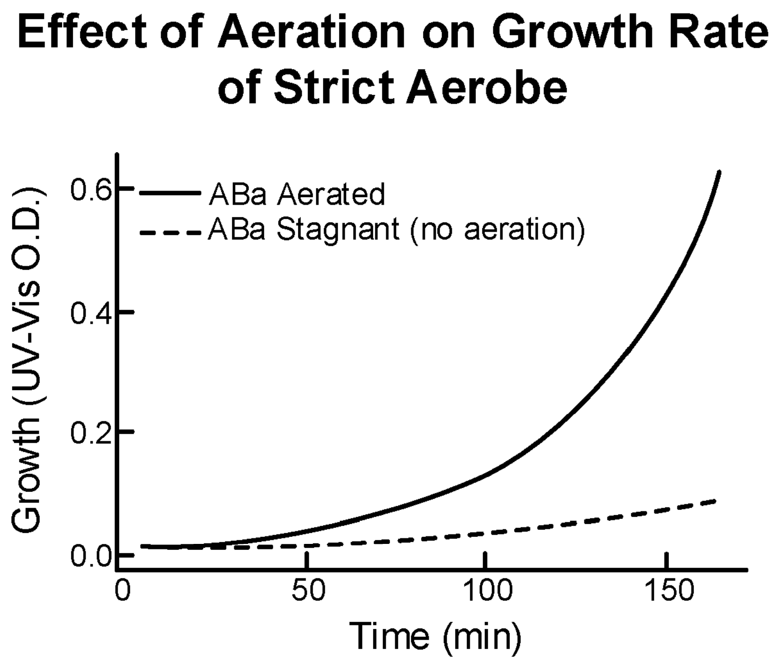
Effect of Aeration on Growth Rate
of Strict Aerobe
ABa Aerated
ABa Stagnant (no aeration)
Growth (UV-Vis O.D.)
0.0
0.2
0.4
0.6
0
50
100
150
Time (min)

FIG. 7B

| | Species | Doubling Time in Minutes Aerated | Stagnant | % Change |
|---|---|---|---|---|
| Facultative anaerobe | Eco | 24.8 | 29.1 | 15 |
| | SMa | 26.3 | 33.3 | 21 |
| | PVu | 27.2 | 32.7 | 17 |
| Strict aerobe | ABa | 30.5 | 51.9 | 41 |
| | PAe | 33.1 | 207.4 | 84 |
| | Coefficient of variation (CV) | 12% | 109% | |

FIG. 8A

| Species | Doubling Time in Minutes Aerated |
|---|---|
| ECo | 24.8 |
| SMa | 26.3 |
| PVu | 27.2 |
| ABa | 30.5 |
| PAe | 33.1 |
| Average Doubling Time ($t_{doubling_average}$): | 28.4 |

FIG. 8B

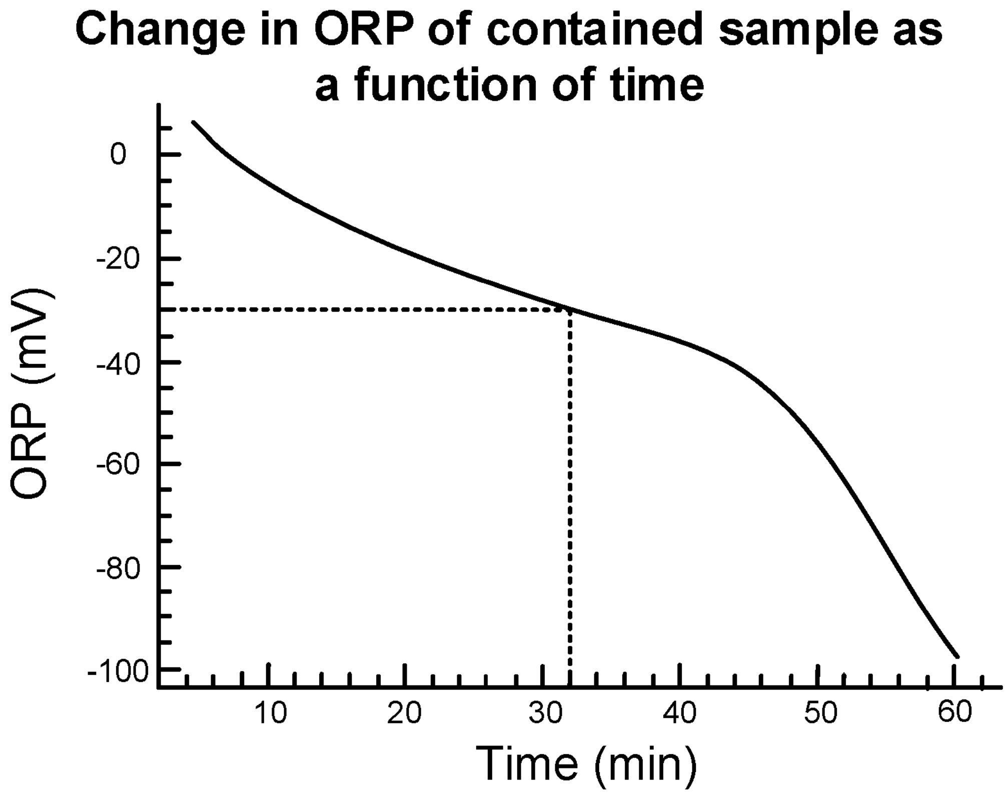
Change in ORP of contained sample as a function of time
ORP (mV)
0
-20
-40
-60
-80
-100
10
20
30
40
50
60
Time (min)

FIG. 9A

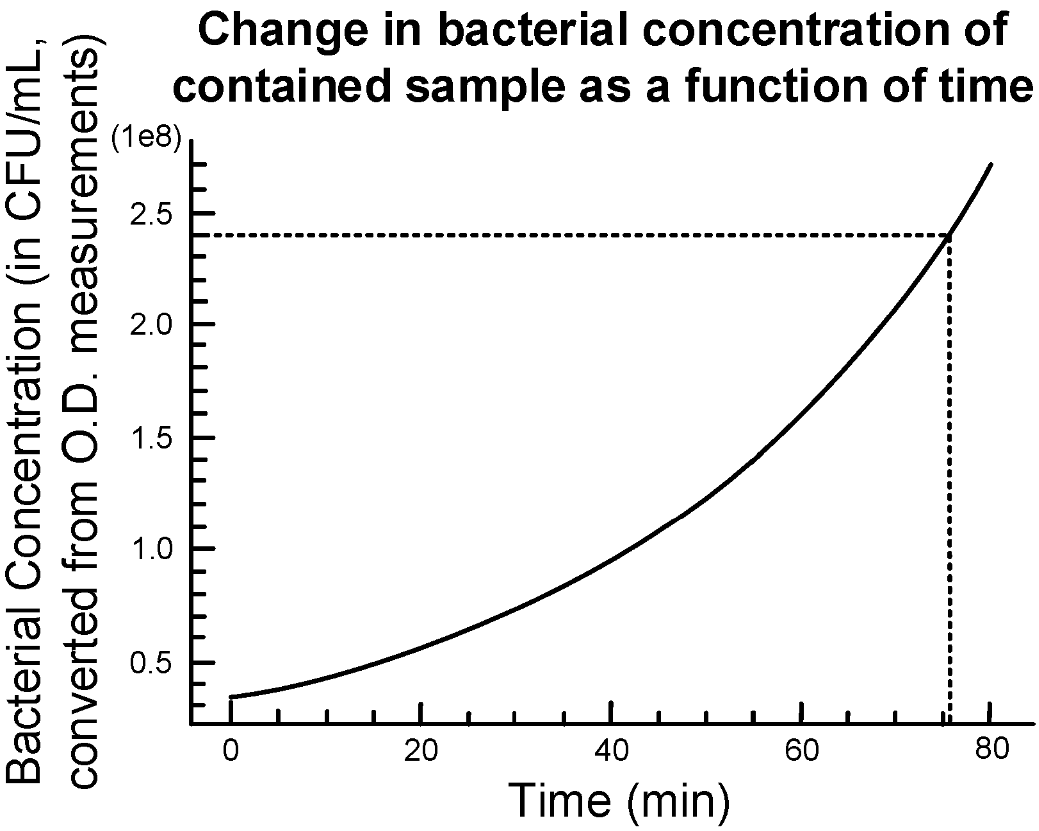
Change in bacterial concentration of contained sample as a function of time
Bacterial Concentration (in CFU/mL, converted from O.D. measurements)
(1e8)
2.5
2.0
1.5
1.0
0.5
0
20
40
60
80
Time (min)

FIG. 9B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2021371896 A1
- WO 2019246208 A1
- US 2019293529 A1
- CN 110713919 A
- WO 2014207532 A1
- US 2013330768 A1
- US 2011081713 A1
- WO 03006629 A1